# FASCICULE DE BREVET EUROPEEN

(11) **EP 3 917 641 B1**
(45) Date de publication et mention de la délivrance du brevet: **08.05.2024**
(21) Numéro de dépôt: 20701203.0
(22) Date de dépôt: 27.01.2020
(51) Int. Cl.: B01D 11/00, C07C 67/54, C07C 69/82, C07C 51/43, C08J 11/04, C08G 63/02, C08G 63/18, C08G 63/78

(54) **PROCÉDÉ DE PRODUCTION D'UN POLYESTER TÉRÉPHTALATE INTÉGRANT UN PROCÉDÉ DE DÉPOLYMÉRISATION**
VERFAHREN ZUR HERSTELLUNG EINES POLYESTERTEREPHTHALATS MIT EINEM DEPOLYMERISATIONSVERFAHREN
METHOD FOR PRODUCING A POLYESTER TEREPHTHALATE INCORPORATING A DEPOLYMERIZATION METHOD

(30) Priorité: 01.02.2019 FR 1901024
(43) Date de publication de la demande: 08.12.2021
(73) Titulaire: IFP Energies nouvelles, 92852 Rueil-Malmaison (FR)
(72) Inventeur: THINON, Olivier, 92852 Rueil-Malmaison (FR); GAUTHIER, Thierry, 92852 Rueil-Malmaison (FR)
(74) Mandataire: IFP Energies nouvelles
(86) Numéro de dépôt international: PCT/EP2020/051844
(87) Numéro de publication internationale: WO 2020/156965

(56) Documents cités:
- EP-A1- 0 723 951
- EP-A1- 1 120 394
- EP-A1- 1 234 812
- EP-A1- 1 306 364
- WO-A1-2016/096768
- WO-A1-2017/006217
- WO-A1-2018/007356
- FR-A1- 2 046 924
- GB-A- 776 282
- GB-A- 931 314
- JP-A- 2003 306 603
- US-A- 4 001 187
- US-A- 5 502 247
- US-A- 5 504 121
- US-A- 5 869 543
- US-A1- 2004 147 624
- US-A1- 2004 182 782
- US-A1- 2006 074 136
- US-A1- 2015 105 532

## Description

### Domaine technique

La présente description concerne un procédé de production d'un polyester, en particulier d'un polyéthylène téréphtalate (PET), intégrant un procédé de dépolymérisation d'une charge polyester à recycler, en particulier comprenant du polyester opaque.

### Technique antérieure

Le recyclage chimique de polyester, en particulier du polyéthylène téréphtalate (PET), a fait l'objet de nombreux travaux visant à décomposer le polyester récupéré sous forme de déchets en monomères qui pourront de nouveau être utilisés comme charge d'un procédé de polymérisation.

De nombreux polyesters sont issus de circuits de collecte et de tri de matières. En particulier, le polyester, en particulier le PET, peut provenir de la collecte de bouteilles, barquettes, films, résines et/ou fibres composées de polyester (comme par exemple des fibres textiles, des fibres de pneus). Le polyester issu de filières de collecte et de tri est appelé polyester à recycler.

Le PET à recycler peut être classé en quatre grandes catégories :
- le PET clair, constitué majoritairement de PET transparent incolore (en général au moins 60% poids) et de PET transparent coloré azuré, qui ne contient pas de pigments et peut être engagé dans des procédés de recyclage mécanique,
- le PET foncé, ou coloré (vert, rouge,..), qui peut contenir généralement jusqu'à 0,1% poids de colorants ou pigments mais reste transparent, ou translucide ;
- le PET opaque, qui contient une quantité significative de pigments à des teneurs variant typiquement entre 0,25 et 5,0% poids pour opacifier le polymère. Le PET opaque est utilisé de manière grandissante par exemple pour la fabrication de contenants alimentaires, comme les bouteilles de lait, dans la composition de flacons cosmétiques, phytosanitaires ou de colorants ;
- le PET multicouches, qui comporte des couches de polymères autres que le PET ou une couche de PET recyclé entre des couches de PET vierge (c'est-à-dire PET n'ayant pas subi de recyclage), ou un film d'aluminium par exemple. Le PET multicouches est utilisé après thermoformage pour faire des emballages tels que des barquettes.

Les filières de collecte permettant d'alimenter les filières de recyclage sont structurées différemment en fonction des pays. Elles évoluent de manière à maximiser la quantité de plastique valorisé dans les déchets en fonction de la nature et de la quantité des flux et des technologies de tri. La filière de recyclage de ces flux est en général constituée d'une première étape de conditionnement sous forme de paillettes au cours de laquelle des balles d'emballage brut sont lavées, purifiées et triées, broyées puis de nouveau purifiées et triées pour produire un flux de paillettes contenant en général moins de 1% massique d'impuretés « macroscopiques » (verre, métaux, autres plastiques, bois, papier carton, éléments minéraux), préférentiellement moins de 0,2% d'impuretés « macroscopiques » et encore plus préférentiellement moins de 0,05%.

Les paillettes de PET clair peuvent ensuite subir une étape d'extrusion-filtration permettant de produire des extrudés qui sont ensuite réutilisables en mélange avec du PET vierge pour faire de nouveaux produits (bouteilles, fibres, films). Une étape de polymérisation sous vide à l'état solide (connu sous l'acronyme SSP) est nécessaire pour les usages alimentaires. Ce type de recyclage est appelé recyclage mécanique.

Les paillettes de PET foncé (ou coloré) sont également recyclables mécaniquement. Cependant, la coloration des extrudés formés à partir des flux colorés limite les usages : le PET foncé est le plus souvent utilisé pour produire des fibres ou des lanières d'emballage. Les débouchés sont donc plus limités par rapport à ceux du PET clair.

La présence de PET opaque contenant des pigments à des teneurs importantes, dans le PET à recycler, pose des problèmes aux recycleurs car le PET opaque altère les propriétés mécaniques du PET recyclé. Le PET opaque est actuellement collecté avec le PET coloré et se retrouve dans le flux de PET coloré. Compte tenu du développement des usages du PET opaque, les teneurs en PET opaque dans le flux de PET coloré à recycler sont actuellement comprises entre 5-20% poids et ont tendance à augmenter encore. D'ici quelques années, il sera possible d'atteindre des teneurs en PET opaque dans le flux de PET coloré supérieures à 20-30% poids. Or il a été montré qu'au-delà de 10-15% de PET opaque dans les flux de PET coloré, les propriétés mécaniques du PET recyclé sont altérées (cf. « *Impact du développement du PET opaque blanc sur le recyclage des emballages en PET* », note préliminaire du COTREP du 5/12/13) et empêchent le recyclage sous forme de fibres, principal débouché de la filière pour le PET coloré.

Les colorants sont des substances naturelles ou synthétiques, solubles notamment dans la matière polyester et utilisés pour colorer la matière dans laquelle ils sont introduits. Les colorants généralement utilisés sont de différentes natures et contiennent souvent des hétéroatomes de type O et N, et des insaturations conjuguées, comme par exemple des fonctions quinone, methine, azo, ou des molécules comme la pyrazolone et la quinophtalone.

Les pigments sont des substances finement divisées, insolubles en particulier dans la matière polyester, utilisés pour colorer et/ou opacifier la matière dans laquelle ils sont introduits. Les principaux pigments utilisés pour colorer et/ou opacifier les polyesters, en particulier le PET, sont des oxydes métalliques comme TiO₂,CoAl₂O₄, Fe₂O₃, des silicates, des polysulfides, et du noir de carbone. Les pigments sont des particules de taille comprise généralement entre 0,1 et 10 µm, et en majorité entre 0,4 et 0,8 µm. L'élimination totale de ces pigments par filtration, nécessaire pour envisager un recyclage du PET opaque, est techniquement difficile car ils sont extrêmement colmatants.

Le recyclage des PET colorés et opaques est donc extrêmement délicat.

Plusieurs procédés de recyclage chimique de polyester, en particulier du PET ont été proposés dans la littérature mais n'adressent pas tous le même produit final, produit qui est ensuite utilisé pour fabriquer à nouveau un polymère d'ester téréphtalate.

En particulier, la demande de brevet MX 2007/004429 divulgue la production d'un polyester de bonne qualité, comprenant un procédé de dépolymérisation par glycolyse à pression atmosphérique de paillettes de PET en présence d'éthylène glycol dans une base de bis(2-hydroxyéthyl) téréphtalate (BHET). Le produit intermédiaire obtenu à l'issue de l'étape de dépolymérisation est filtré sur un système de fritté pour retenir des particules d'au moins 25µm avant d'être introduit dans le réacteur de polymérisation, pour obtenir un polyester de bonne qualité. Dans le brevet US 4,578,502, des monomères d'acide carboxylique sous forme cristallisé et de polyol sous forme liquide sont récupérés après dépolymérisation d'une résine polyalkylène téréphtalate par hydrolyse ou méthanolyse, puis recombinés pour reformer du polyester. La demande de brevet WO 2013/025186 décrit quant à elle un procédé de préparation d'un copolyester ayant une forte teneur de monomères recyclée, comprenant une étape de dépolymérisation par méthanolyse, une étape de séparation du diméthyl téréphtalate libéré puis la polymérisation du diméthyl téréphtalate avec au moins un polyol.

La demande de brevet US 2006/0074136 décrit un procédé de dépolymérisation par glycolyse de PET coloré, en particulier issu de la récupération de bouteilles de PET colorées vertes. Le flux de BHET obtenu à l'issue l'étape de glycolyse est purifié sur charbon actif pour séparer certains colorants, comme les colorants bleu, puis par extraction des colorants résiduels, comme les colorants jaune, par un alcool ou par l'eau. Le BHET qui cristallise dans le solvant d'extraction est alors séparé, dans le but de pouvoir être utilisé dans un procédé de polymérisation de PET. Dans la demande de brevet US 2015/0105532, le PET post-consommation comprenant un mélange différents PET colorés comme le PET clair, le PET bleu, le PET vert et/ou le PET ambré, est dépolymérisé par glycolyse en présence d'un catalyseur amine et d'alcool. Le monomère diester alors obtenu peut être purifié par filtration, échange d'ions et/ou passage sur charbon actif, avant d'être cristallisé et récupéré par filtration pour pouvoir être polymérisé et ainsi reformer un polyester.

La demande de brevet WO 2017/006217 divulgue le procédé de préparation d'un polyéthylène téréphtalate glycol modifié (r-PETG) comprenant une étape de dépolymérisation d'un PET en présence d'un mélange de monoéthylène glycol (MEG) et de neopentyl glycol, suivie directement d'une étape de polymérisation de l'effluent de réaction.

La demande de brevet FR 3053691 et la demande WO 2018/007356 décrient un procédé de dépolymérisation d'une charge polyester comprenant en particulier de 0,1 à 10% poids de pigments, par glycolyse en présence d'éthylène glycol. Un effluent de monomères bis-(2-hydroxyethyl) téréphtalate (BHET), obtenu après des étapes particulières de séparation et de purification, peut alimenter une étape de polymérisation en vue de produire du PET, sans qu'aucune condition ne soit préciser. Dans le brevet EP0865464, le procédé de dépolymérisation de polyesters, dont la nature n'est pas précisée, comprend les étapes de dépolymérisation en présence d'un diol, d'évaporation du diol, de dissolution du mélange dans un solvant chaud, de filtration et de précipitation de la solution filtrée, le précipité pouvant ensuite servir pour la préparation d'un nouveau polymère.

Le brevet JP3715812 décrit l'obtention de BHET raffiné à partir de PET, le BHET obtenu pouvant être utilisé comme matière première dans un procédé de production de produits en matière plastique. La dépolymérisation est suivie d'une étape de pré-purification par refroidissement, filtration, adsorption et traitement sur résine échangeuse d'ions, présentée comme très importante, réalisée avant l'évaporation du glycol et la purification du BHET. La pré-purification permet d'éviter la re-polymérisation du BHET dans les étapes subséquentes de purification. Cependant, le passage par une étape de filtration et résine échangeuse d'ions peut être extrêmement problématique lorsque la charge comprend une quantité importante de très petites particules solides, à l'instar des pigments, ce qui est le cas lorsque la charge traitée comprend du PET opaque, en particulier en proportions conséquentes (plus de 10% poids de PET opaque). Parallèlement, le brevet EP 1 120 394 divulgue un procédé de production de BHET et/ou de ses oligomères (« low condensate thereof ») à partir d'un polyester aromatique et l'utilisation du BHET obtenu comme matière première pour la re-production d'un polyester de haute qualité de bis-(2-hydroxyethyl) téréphtalate de haute pureté. Pour cela, le brevet EP 1 120 394 décrit un procédé de dépolymérisation d'un polyester comprenant une étape de glycolyse en présence d'éthylène glycol et un procédé de purification d'une solution de bis-(2-hydroxyethyl) téréphtalate sur une résine échangeuse de cations et une résine échangeuse d'anions.

Le brevet US 5,869,543 divulgue un procédé de préparation de polyéthylène téréphtalate à partir de déchets de polyéthylène téréphtalate, ledit procédé comprenant une étape g) de mélange de cristaux de BHET avec entre 0,2 et 1 fois la quantité molaire d'acide téréphtalique. Le procédé comprend ensuite une étape de réaction du mélange obtenu.

Le brevet US 4,001,187 divulgue un procédé de production de polyéthylène téréphtalate à partir d'un « slurry », correspondant à une suspension d'acide téréphtalique dans de l'éthylène glycol, qui alimente en continu un système réactionnel contenant du BHET et des oligomères. Aucune étape de dépolymérisation de polyester n'est divulguée dans US 4,001,187.

La demande de brevet JP2003/306603 divulgue un procédé de préparation de polyester à partir d'un slurry d'acide téréphtalique et d'éthylène glycol introduit en continu dans le système réactionnel à 250°C. Même si le système réactionnel contient probablement du BHET, JP2003/306603 ne divulgue pas d'étape de mélange de l'acide téréphtalique avec une charge monomère diester comprenant du BHET. Aucune étape de dépolymérisation de polyester et de séparation-purification d'un effleunt diester comprenant du BHET n'est décrite dans J P2003/306603.

Le document GB 776,282 divulgue la préparation de BHET à partir d'acide téréphtalique et d'éthylène glycol, dans un rapport molaire de 1 : 6, 1 : 4 ou 1 : 2, à la température d'ébullition de l'éthylène glycol, c'est-à-dire à environ 197°C (l'acide téréphtalique étant solide à pression ambiante et se sublimant à 402°C), de préférence en mélange avec du BHET préalablement formé par réaction de l'acide téréphtalique avec l'éthylène glycol.

Le document FR 2046924 divulgue un procédé de production de PET directement à partir de l'acide téréphtalique et d'éthylène-glycol, le mélange étant chauffé à 260°C ou plus. Aucune étape de dépolymérisation de polyester et de séparation-purification d'un effleunt diester comprenant du BHET n'est décrite dans FR 2046924.

Le brevet US 5,502,247 divulgue un procédé de dépolymérisation de polyester par hydrolyse pour obtenir des monomères acide téréphtalique et éthylène glycol.

Le brevet US 5,504,121 divulgue un procédé de dépolymérisation du PET pour obtenir de l'acide téréphtalique et de l'éthylène glycol, qui peuvent éventuellement être repolymérisés
D'autres documents, comme EP 0723951, WO 2016/096768, US 2004/0147624, EP 1306364, EP 1234812, US 2004/0182782, ou encore GB931314, divulguent des méthodes de purification du bis(hydroxyéthylène) téréphtalate (BHET), obtenu après dépolymérisation de charges polyester, par filtration, décantation, centrifugation, extraction, distillation, (re)cristallisation, par traitement avec un décolorant (charbon active) et/ou des résines d'échange d'ions.

Aucun de ces documents ne propose l'enchainement direct des étapes de dépolymérisation, en particulier de PET à recycler comprenant du PET foncé et/ou opaque, et de polymérisation des produits intermédiaires obtenus, avec une intégration optimisée notamment des flux de matières, en particulier des flux de diol, permettant une réduction conséquente des consommations en matières premières et en énergie.

### Résumé de l'invention

L'invention a pour objet un procédé de production d'un polyester téréphtalate à partir d'au moins une charge polyester à recycler comprenant au moins 10% poids de PET opaque, comprenant au moins les étapes suivantes :
a) une étape de dépolymérisation de ladite charge polyester à recycler, comprenant au moins une section réactionnelle alimentée en ladite charge polyester à recycler et en une charge glycol comprenant au moins une fraction d'un flux diol purifié, ladite section réactionnelle étant opérée, à une température comprise entre 150 et 400°C, de préférence entre 180 et 300°C, de manière préférée entre 200°C et 280°C, à une pression d'au moins 0,1 MPa, préférentiellement au moins 0,4 MPa, et avec un temps de séjour par réacteur compris entre 0,05 et 10 h, pour obtenir un effluent réactionnel de dépolymérisation,
b) une étape de séparation, comprenant au moins une section de séparation alimentée en ledit effluent réactionnel de dépolymérisation obtenu à l'issue de l'étape a) de dépolymérisation, pour obtenir au moins un effluent glycol et un effluent diester,
c) une étape de purification de l'effluent diester obtenu à l'issue de l'étape b), comprenant au moins une section de séparation alimentée par ledit effluent diester obtenu à l'issue de l'étape b) et opérée à une température inférieure ou égale à 250°C, à une pression inférieure ou égale à 0,001 MPa, et avec un temps de séjour liquide par section inférieur ou égal à 10 min, puis une section de décoloration opérée à une température entre 100 et 250°C et à une pression entre 0,1 et 1,0 MPa, en présence d'un adsorbant, pour obtenir un effluent diester purifié liquide comprenant au moins du bis(2-hydroxyéthyl) téréphtalate (BHET),
d) une étape de préparation d'une charge de polymérisation comprenant au moins une section de mélange alimentée en au moins une charge téréphtalique, qui comprend de l'acide téréphtalique ou du diméthyle téréphtalate, et au moins une fraction dudit effluent diester purifié liquide obtenu à l'étape c), les quantités en au moins ladite charge téréphtalique et ladite fraction de l'effluent diester purifié liquide, introduites dans ladite section de mélange, étant ajustées de sorte que le rapport du nombre total de moles de motifs diol de formule - [C₍ₙ₊₁₎H₍₂ₙ₊₂₎O₂]-, n étant un nombre entier supérieur ou égal à 1, introduits dans ladite section de mélange, par rapport au nombre total de moles de motifs téréphtalate de formule -[CO-(C₆H₄)-CO]-, introduits dans ladite section de mélange, est compris entre 1,0 et 2,0, ladite section de mélange étant opérée à une température comprise entre 100 et 150°C et à une pression supérieure ou égale à 0,1 MPa,
e) une étape de condensation de ladite charge de polymérisation issue de l'étape d), pour produire au moins un effluent réactionnel de condensation, un effluent diol et un effluent aqueux ou un effluent méthanol, ladite étape de condensation comprenant au moins une section réactionnelle opérée à une température entre 150 et 400°C, à une pression entre 0,05 et 1 MPa, et avec un temps de séjour entre 1 et 10 h, et au moins une section de séparation,
f) une étape de polycondensation dudit effluent réactionnel de condensation obtenu à l'étape e) pour obtenir au moins ledit polyester téréphtalate et un effluent diol, ladite étape de polycondensation comprenant au moins une section réactionnelle comprenant au moins un réacteur dans lequel est mise en oeuvre la polycondensation et étant opérée à une température entre 200 et 400°C, à une pression entre 0,0001 et 0,1 MPa, avec un temps de séjour entre 0,1 et 5 h, ladite section réactionnelle comprenant également au moins un soutirage dudit effluent diol,
g) une étape de traitement des diols, comprenant une section de récupération alimentée au moins par tout ou partie de l'effluent glycol issu de l'étape b) et tout ou partie de l'effluent diol issu de l'étape f), pour obtenir un flux diol à traiter, et une section de purification dudit flux diol à traiter pour obtenir le flux diol purifié qui est envoyé en tout ou partie vers l'étape a).

De préférence, la présente invention concerne un procédé de production d'un polyester téréphtalate à partir d'au moins une charge polyester à recycler consistant en les étapes a), b), c), d), e), f) et g), décrites ci-dessus.

Un intérêt majeur du procédé de production d'un polyester téréphtalate réside dans la production d'un polyester à partir au moins en partie de composé issu de polyester à recycler, le polyester à recycler comprenant notamment du polyester opaque, en quantité non négligeable et conforme à celle rencontrée dans la matière polyester issue des filières de collecte et de tri, sans détérioration de la qualité du polyester produit. En effet, le présent procédé permet la production d'un polyester à partir au moins en partie de polyester à recycler et qui présente des propriétés, notamment physico-chimiques et mécaniques, similaires à celles d'un polyester vierge de même nature.

Le présente procédé qui comprend un enchainement d'un procédé de dépolymérisation en particulier par glycolyse d'une matière polyester à recycler et d'un procédé de polymérisation d'un polyester permet une intégration de flux de matières. Il permet plus particulièrement, notamment par un système de recyclage spécifique, une intégration optimisée des flux diol introduits et récupérés entre les phases de dépolymérisation et de polymérisation. L présent procédé permet également la production d'un intermédiaire diester purifié liquide qui répond aux spécifications du procédé de polymérisation et qui peut donc directement alimenter les étapes de polymérisation sans étape intermédiaire de purification et/ou de conditionnement, comme par exemple la cristallisation dudit intermédiaire puis le passage à une phase liquide dudit intermédiaire diester cristallisé, ce qui permet d'éviter des manipulations supplémentaires et une consommation d'énergie importante. Parallèlement, l'incorporation d'un intermédiaire diester purifié obtenu sous forme liquide à l'issue des étapes de dépolymérisation, dans les étapes de polymérisation, en particulier dans le mélange des monomères, permet de diminuer la quantité de la charge de monomère diol alimentant le procédé de polymérisation, tout en conservant une bonne opérabilité notamment de l'étape de préparation du mélanges des monomères du procédé de polymérisation. La quantité de diol introduite dans le mélange étant réduite, la quantité de diol à recycler dans les opérations ultérieures du procédé de polymérisation est diminuée, réduisant ainsi la consommation énergétique du procédé global de production d'un polyester à partir au moins en partie de matière polyester à recycler.

Un autre avantage est la mutualisation d'opérations de récupération et de purification des effluents diol issus du procédé de dépolymérisation et du procédé de polymérisation, induisant de fait une réduction des consommations énergétiques du procédé global et des coûts d'équipements diminués.

### Description des modes de réalisation

La présente descriptionconcerne un procédé de production d'un polyester intégrant un procédé de dépolymérisation d'une charge polyester à recycler, en particulier comprenant du polyester opaque.

Selon la présente description, les termes « polyester », « polyester téréphtalate » et « polyalkylène téréphtalate » sont interchangeables et désignent un polymère (c'est-à-dire une molécule de masse moléculaire élevée constituée de monomères unis les uns aux autres par des liaisons covalentes) dont le motif élémentaire de répétition comprend un motif téréphtalate -[CO-(C₆H₄)-CO]-, où -(C₆H₄)- représente un cycle aromatique, lié de façon covalente à un motif diol -[O-C₍ₙ₊₁₎H₍₂ₙ₊₂₎-O]-, où n est un nombre entier supérieur ou égal à 1, de préférence comprend entre 1 et 5, préférentiellement entre 1 et 3. Très classiquement, un polyalkylène téréphtalate est le résultat de la polycondensation d'un monomère diol (ou glycol) avec un monomère acide téréphtalique (ou diméthyle téréphtalate). Le polyester téréphtalate, ou polyalkylène téréphtalate est, en particulier, le polyéthylène téréphtalate (PET), le polybutylène téréphtalate (PBT), le polytriméthylène téréphtalate (PTT) ou tout autre polymère dont le motif de répétition de la chaine principale contient une fonction ester et un cycle aromatique issu de l'acide téréphtalique (ou d'un de ses esters, en particulier du téréphtalate de diméthyle). Le polyester téréphtalate préféré est le polyéthylène téréphtalate ou poly(téréphtalate d'éthylène), nommé encore simplement PET, dont le motif élémentaire de répétition présente la formule suivante :

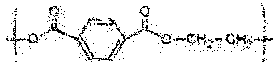

Classiquement, le PET est obtenu par polycondensation de l'acide téréphtalique (PTA), ou du diméthyle téréphtalate (DMT), avec l'éthylène glycol.

Selon la présente description, l'expression « à recycler » qualifie toute matière, en particulier comprenant du polyester, issue des filières de collecte et de tri des déchets plastiques. Par opposition, un polyester vierge est uniquement issu de la polymérisation de charges monomères comprenant au moins un acide dicarboxylique (par exemple l'acide téréphtalique, PTA) ou un ester dicarboxylate (par exemple le téréphtalate de diméthyle, DMT) et au moins un composé de la famille des diols ou glycols (par exemple l'éthylène glycol).

Selon la présente description, le terme « monomère diester » désigne un composé d'ester téréphtalate de formule chimique HOC₍ₘ₊₁₎H₍₂ₘ₊₂₎-CO₂-(C₆H₄)-CO₂-C₍ₙ₊₁₎H₍₂ₙ₊₂₎OH, dans laquelle : -(C₆H₄)- représente un cycle aromatique ; n et m sont des nombres entiers, identiques ou différents, de préférence identiques (c'est-à-dire n = m), et supérieurs ou égaux à 1, de préférence compris entre 1 et 5, de manière préférée compris entre 1 et 3. Une molécule de monomère diester correspond à un composé qui résulterait de l'estérification d'une molécule d'acide téréphtalique HOOC-(C₆H₄)-COOH (où -(-(C₆H₄)- représentant un cycle aromatique) avec deux molécules d'au moins un diol (ou glycol), plus particulièrement avec une molécule d'un diol de formule chimique HO-C₍ₙ₊₁₎H₍₂ₙ₊₂₎-OH et une molécule d'un diol de formule chimique HO-C₍ₘ₊₁₎H₍₂ₘ₊₂₎-OH. Le monomère diester préféré est le bis(2-hydroxyéthyl) téréphtalate (BHET).

Le terme oligomère désigne typiquement un polymère de petite taille, constitué généralement de 2 à 20 motifs élémentaires de répétition.

Selon la présente description, le terme « oligomère d'ester » désigne un oligomère d'ester téréphtalate comprenant entre 2 et 20, de préférence entre 2 et 5, motifs élémentaires de répétition de formule -[O-CO-(C₆H₄)-CO-O-C₍ₙ₊₁₎H₍₂ₙ₊₂₎]-, avec : -(C₆H₄)- un cycle aromatique et n un nombre entier supérieur ou égal à 1, de préférence compris entre 1 et 5, de manière préférée entre 1 et 3.

Par « colorant », on entend une substance soluble dans la matière polyester et utilisée pour la colorer. Le colorant peut être d'origine naturelle ou synthétique.

Par « pigment », plus particulièrement pigment colorant et/ou opacifiant, on entend une substance finement divisée, insoluble dans la matière polyester. Les pigments sont sous forme de particules de taille comprise généralement entre 0,1 et 10 µm, et en majorité entre 0,4 et 0,8 µm. Ils sont souvent de nature minérale. Les pigments généralement utilisés, notamment pour opacifier, sont des oxydes métalliques comme TiO₂, CoAl₂O₄, Fe₂O₃, des silicates, des polysulfides, et du noir de carbone.

Selon la présente description, l'expression « compris entre ... et ... » signifie que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente description.

### Charges

Le procédé est alimenté en au moins une charge polyester à recycler.

Avantageusement, ladite charge polyester à recycler est issue des filières de collecte et de tri des déchets, en particulier des déchets plastiques. Ladite charge polyester à recycler peut provenir, par exemple, de la collecte de bouteilles, barquettes, films, résines et/ou fibres constitués de polyéthylène téréphtalate.

Ladite charge polyester à recycler peut être, en tout ou partie, sous forme de paillettes (ou flakes selon le terme anglais), dont la plus grande longueur est inférieure à 10 cm, préférentiellement comprise entre 5 et 25 mm ou sous forme de solide micronisé c'est-à-dire sous de particules de préférence ayant une taille comprise entre 10 micron et 1 mm. La charge polyester à recycler comprend de préférence moins de 2% poids, préférentiellement moins de 1 % poids d'impuretés « macroscopiques » comme du verre, du métal, du plastique autre que polyester téréphtalate, du bois, du papier carton, des éléments minéraux. Ladite charge polyester à recycler peut également être, en tout ou partie, sous forme de fibres, telles que des fibres textiles, éventuellement prétraitées pour éliminer des fibres de coton, de polyamide, ou tout autre fibre textile autre que polyester, ou telles que des fibres de pneus, éventuellement prétraitées pour éliminer notamment des fibres polyamide ou des résidus de caoutchouc ou de polybutadiène. Ladite charge polyester à recycler peut, en outre, comprendre du polyester issu des rebuts de production des procédés de polymérisation et/ou de transformation de la matière polyester.

Avantageusement, ladite charge polyester à recycler contient plus de 50 % masse de polyalkylène téréphtalate, de préférence plus de 70% poids, de manière préférée plus 90 % poids de polyalkylène téréphtalate.

De préférence, ladite charge polyester à recycler est une charge polyéthylène téréphtalate à recycler (ou charge PET à recycler) qui comprend plus de 50 % masse de polyéthylène téréphtalate (PET), de préférence plus de 70% poids de polyéthylène téréphtalate (PET), de manière préférée plus 90 % poids de polyéthylène téréphtalate (PET). Ladite charge PET à recycler comprend avantageusement du PET opaque, foncé (ou coloré), multicouche, clair (c'est-à-dire transparent incolore et/ou transparent coloré azuré) et leurs mélanges. de préférence au moins du PET opaque. Elle comprend au moins 10% poids de PET opaque, de manière très préférée au moins 15% poids de PET opaque.

Ladite charge polyester à recycler peut contenir jusqu'à 10% poids de pigments, en particulier entre 0,1% et 10% poids de pigments, notamment entre 0,1 et 5% poids de pigments de pigments, et/ou jusqu'à 1% poids de colorants, en particulier entre 0,05% et 1% poids de colorants, notamment entre 0,05 et 0,2% poids de colorants.

Ladite charge polyester à recycler peut également contenir des éléments utilisés comme catalyseur de polymérisation et/ou comme agents stabilisants dans les procédés de production de polyester, comme l'antimoine, le titane, l'étain.

L'étape a) de dépolymérisation est en outre alimenté en une charge glycol. Ladite charge glycol comprend au moins un, de préférence un, composé glycol (ou diol), de nature identique ou différente que le diol correspondant au motif diol du motif élémentaire du polyalkylène téréphtalate de la charge polyester à recycler. Avantageusement la teneur pondérale de ladite charge glycol en ledit composé glycol est pondérale supérieure ou égale à 80% poids, préférentiellement supérieure ou égale à 90% poids, de manière préférée supérieure ou égale à 95% poids, par rapport au poids total de ladite charge glycol.

De préférence, la charge glycol du procédé qui alimente l'étape a) de dépolymérisation comprend l'éthylène glycol, nommé aussi monoéthylène glycol (MEG), avantageusement à une teneur pondérale supérieure ou égale à 80% poids, préférentiellement supérieure ou égale à 90% poids, de manière préférée supérieure ou égale à 95% poids, par rapport au poids total de ladite charge glycol. Dans ce cas-là, la charge glycol est appelé charge éthylène glycol.

L'éthylène glycol peut être avantageusement produit par hydrolyse de l'oxyde d'éthylène ou par hydrogénation sélective de glycolaldéhyde ou dépolymérisation de polyesters ou par tout autre procédé permettant d'obtenir une charge éthylène glycol avec les spécifications requises par les procédés de polymérisation. L'éthylène glycol peut être issu de sources hydrocarbures fossiles ou de la biomasse.

Ladite charge glycol qui alimente l'étape a) de dépolymérisation du procédé comprend, de préférence consiste en, au moins une fraction dudit flux diol purifié obtenu à l'issue de l'étape g) du procédé de production du polyester téréphtalate.

Ledit procédé est également alimenté, à l'étape d) de préparation d'une charge de polymérisation, en au moins une charge téréphtalique. Selon la présente description, une charge téréphtalique est une charge acide téréphtalique, qui comprend l'acide téréphtalique comme composé à motif téréphtalate, ou une charge diméthyle téréphtalate, qui comprend le diméthyle téréphtalate comme composé à motif téréphtalate.

Ladite charge téréphtalique, comprend de l'acide téréphtalique (PTA) ou le diméthyle téréphtalate (DMT), à une teneur pondérale supérieure ou égale à 95% poids, préférentiellement supérieure ou égale à 98% poids, de manière préférée supérieure ou égale à 99% poids, par rapport au poids total de ladite acide téréphtalique.

Dans un mode de réalisation préféré, ladite charge est une charge téréphtalique qui comprend de l'acide téréphtalique (PTA). L'acide téréphtalique de la charge acide téréphtalique peut avantageusement être produit par oxydation du para-xylène ou par dépolymérisation de polyesters ou par tout autre procédé permettant d'obtenir une charge d'acide téréphtalique avec les spécifications requises par les procédés de polymérisation. L'acide téréphtalique peut être issu de sources hydrocarbures fossiles ou de la biomasse.

La charge acide téréphtalique est avantageusement sous forme de poudre, c'est-à-dire sous forme de particules solides d'acide téréphtalique. Les particules d'acide téréphtalique incorporées dans le mélange de monomères présentent, de préférence, un diamètre moyen de préférence compris entre 1 et 1000 µm, en particulier entre 30 et 500 µm et notamment entre 80 et 200 µm. Le diamètre moyen des particules d'acide téréphtalique est déterminé par toute méthode d'analyse granulométrique connue de l'Homme du métier, comme par exemple par diffraction laser ou par tamisage, de préférence par tamisage sur une colonne de tamis adaptés selon une technique connue de l'Homme du métier.

### Etape a) de dépolymérisation

Le procédé de production d'un polyester téréphtalate comprend une étape a) de dépolymérisation d'une charge polyester à recycler, pour obtenir un effluent réactionnel de dépolymérisation. Ladite étape a) de dépolymérisation comprend au moins une section réactionnelle qui est alimentée en ladite charge polyester à recycler et en une charge glycol.

Avantageusement, l'alimentation en ladite charge glycol est ajustée de sorte que la quantité du composé glycol contenu dans ladite charge glycol correspond, en entrée de ladite section de réactionnelle, à 1 à 20 moles, de préférence de 3 à 10 moles, de composé glycol par mole de motif élémentaire de répétition du polyester contenu dans ladite charge polyester à recycler.

Ladite charge glycol comprend, de préférence consiste en, au moins une fraction dudit flux diol purifié obtenu à l'issue de l'étape g) du procédé de production du polyester téréphtalate.

Avantageusement, l'étape a) de dépolymérisation met en oeuvre, en particulier dans ladite section réactionnelle, une réaction de glycolyse du polyalkylène téréphtalate de ladite charge polyester à recycler en présence du(des) composé(s) glycol de ladite charge glycol.

Ladite section réactionnelle de l'étape a) de dépolymérisation est opérée, de préférence dans un ou plusieurs réacteurs, à une température comprise entre 150 et 400°C, de préférence entre 180 et 300°C, de manière préférée entre 200°C et 280°C, à une pression d'au moins 0,1 MPa, préférentiellement au moins 0,4 MPa, et avec un temps de séjour par réacteur compris entre 0,05 et 10 h, , préférentiellement entre 0,1 et 6 h et de manière préférée entre 0,5 et 4 h. Le temps de séjour, dans un réacteur de ladite section réactionnelle de l'étape a), est défini comme le rapport du volume de liquide réactionnel dans un réacteur de ladite section réactionnelle sur le débit volumique du flux qui alimente ledit réacteur de ladite section réactionnelle.

Tout type de réacteur connu de l'Homme du métier permettant de réaliser une réaction de dépolymérisation ou de trans-estérification peut être utilisé dans la section réactionnelle de l'étape a). De préférence, le(s) réacteur(s) dans la section réactionnelle de l'étape a) est(sont) agités par un système d'agitation mécanique ou/et par boucle de recirculation ou/et par fluidisation. Ledit(lesdits) réacteur(s) peu(ven)t comprendre un fond conique permettant de purger les impuretés. Avantageusement, ladite section réactionnelle de l'étape a) peut comprendre un(des) réacteur(s) tubulaire(s) ou une association d'un (de) réacteur(s) agité(s) et tubulaire(s) en série ou en parallèle.

Dans un mode de réalisation particulier, l'étape a) de dépolymérisation comprend au moins une section réactionnelle alimentée en une charge PET à recycler et en une charge éthylène glycol, de sorte que la quantité d'éthylène glycol contenu dans ladite charge éthylène glycol correspond à 1 à 20 moles, de préférence de 3 à 10 moles, d'éthylène glycol de ladite charge éthylène glycol par mole de motif élémentaire de répétition c'est à dire de motif téréphtalate dans ladite charge PET à recycler, ladite section réactionnelle étant opérée , dans un ou plusieurs réacteurs, à une température comprise entre 150 et 400°C, de préférence entre 180 et 300°C, de manière préférée entre 200°C et 280°C, à une pression opératoire d'au moins 0,1 MPa, préférentiellement au moins 0,4 MPa, et avec un temps de séjour par réacteur compris entre 0,05 et 10 h de préférence entre 0,1 et 6 h et de manière préférée entre 0,5 et 4 h, ledit temps de séjour par réacteur étant défini comme le rapport du volume liquide du réacteur par le débit volumique du flux qui alimente ledit réacteur.

La réaction de dépolymérisation de l'étape a) peut être réalisée avec ou sans adjonction d'un catalyseur. Lorsque la réaction de dépolymérisation est réalisée après adjonction d'un catalyseur, ce dernier peut être homogène ou hétérogène et choisi parmi les catalyseurs d'estérification connus de l'Homme du métier tels que les complexes oxydes et sels d'antimoine, d'étain, de titane, les alkoxydes de métaux des groupes (I) et (IV) de la classification périodique des éléments, les peroxydes organiques, les oxydes métalliques acido-basiques. De préférence, la réaction de dépolymérisation est réalisée sans adjonction de catalyseur.

La réaction de dépolymérisation peut également être avantageusement réalisée en présence d'un agent adsorbant solide sous forme de poudre ou mis en forme, dont la fonction est de capter au moins une partie des impuretés, en particulier des impuretés colorées, soulageant ainsi la phase de purification de l'étape b). Ledit agent adsorbant solide est en particulier un charbon actif.

L'effluent réactionnel de dépolymérisation obtenu à l'issue de l'étape a) de dépolymérisation comprend un mélange de monomères diester (c'est-à-dire des composés de formule chimique HOC₍ₘ₊₁₎H₍₂ₘ₊₂₎-CO₂-(C₆H₄)-CO₂-C₍ₙ₊₁₎H₍₂ₙ₊₂₎OH, dans laquelle : -(C₆H₄)- représente un cycle aromatique ; n et m sont des nombres entiers, identiques ou différents, de préférence identiques, et supérieurs ou égaux à 1, de préférence entre compris 1 et 5, de manière préférée compris entre 1 et 3), d'oligomères comprenant entre 1 et 5, de préférence entre 1 et 3, motifs élémentaires de formule -[O-CO-(C₆H₄)-CO-O- C₍ₙ₊₁₎H₍₂ₙ₊₂₎]-, avec n un nombre entier compris entre 1 et 5, de préférence entre 1 et 5, de composés diols, d'impuretés éventuellement présentes dans ladite charge polyester à recycler et de composés éventuellement produits à l'issue de réactions secondaires comme par exemple des réactions d'éthérification ou de dégradation. Les composés diols éventuellement compris dans l'effluent réactionnel de dépolymérisation sont avantageusement les monomères et co-monomères diols entrant dans la composition de la charge polyester à recycler et libérés à l'issue de la réaction de dépolymérisation et ceux non réagis issus de la charge glycol alimentant l'étape a) de dépolymérisation. Ledit effluent réactionnel de dépolymérisation peut également contenir des polyesters non convertis et autres polymères.

### Etape b) de séparation

Le procédé de production d'un polyester téréphtalate comprend une étape b) de séparation, comprenant au moins une section de séparation alimentée en ledit effluent réactionnel de dépolymérisation obtenu à l'issue de l'étape a) de dépolymérisation pour obtenir au moins un effluent glycol et un effluent diester.

Ladite étape b) de séparation permet avantageusement de récupérer les composés diol de la charge glycol de l'étape a) n'ayant pas réagis et les composés diols éventuellement libérés au cours de la réaction de dépolymérisation. Ledit effluent glycol obtenu à l'issue de ladite étape b) comprend de préférence au moins 50% poids, préférentiellement au moins 70% poids, de manière préférée plus de 90% poids de composés diols.

Avantageusement, ladite section de séparation comprend une ou plusieurs opérations de séparation, pour permettre la récupération d'un effluent enrichi en diols (nommé effluent glycol), et éventuellement la récupération d'un effluent enrichi en impuretés légères et d'un effluent enrichi en impuretés lourdes. En particulier, la section de séparation peut comprendre une ou plusieurs colonnes de séparation (distillation, stripage, rectification) permettant d'obtenir au moins un effluent enrichi en diols (c'est-à-dire l'effluent glycol), et éventuellement un effluent enrichi en impuretés légères et un effluent enrichi en impuretés lourdes. De préférence, ladite section de séparation comprend une succession de séparations gaz-liquide, de préférence 1 à 5 séparations gaz-liquide, préférentiellement entre 3 et 5 séparations gaz-liquide, opérées à une température comprise entre 100 et 250°C, de préférence entre 110 et 220°C, de manière préférée entre 120 et 210 °C, et à une pression comprise entre 0,00001 et 0,2 MPa, de préférence entre 0,00004 et 0,15 MPa, de manière préférée entre 0,00004 et 0,1 MPa. Les dites séparations gaz-liquide sont avantageusement agitées par toute méthode connue de l'homme du métier. Dans ces conditions de pression et de température, au moins une partie du diol compris dans l'effluent réactionnel, sous forme liquide, est vaporisée à chaque séparation gaz-liquide et séparée d'un flux liquide qui comprend le monomère diester, de telle sorte que la cristallisation du monomère diester et sa polymérisation sont évitées. Avantageusement, la température et la pression de la séparation gaz-liquide ultérieure est inférieure à celle de la séparation gaz-liquide antérieure de manière à ce qu'au moins une partie de l'effluent glycol sortant de la séparation antérieure puisse, en se condensant, rebouillir une partie de l'effluent liquide de la séparation ultérieure. Dans cette configuration, l'apport de chaleur pour récupérer le glycol est minimisé.

Avantageusement, une opération de séparation des différents diols et éventuellement de colorants, alcools légers ou eau, éventuellement compris dans le glycol récupéré par la succession des séparations gaz-liquide, peut être mise en oeuvre dans ladite étape b) de séparation. Ainsi, l'effluent glycol obtenu à l'issue de l'étape b) comprend le composé diol correspondant au motif diol du motif élémentaire du polyalkylène téréphtalate de la charge polyester à recycler, à une teneur supérieure ou égale à 50% poids, préférentiellement supérieure ou égale à 70% poids, de manière préférée supérieure ou égale à 90% poids. De préférence, cette opération éventuelle de séparation est mise en oeuvre dans des colonnes de distillation, de stripage ou de rectification, et avantageusement opérée à une température entre 50 et 250°C, de préférence entre 60 et 210°C, de manière préférée entre 70 et 180°C, et à une pression entre 0,00001 et 0,2 MPa, de préférence entre 0,00004 et 0,15 MPa, de manière préférée entre 0,00004 et 0,1 MPa.

De préférence, tout ou partie dudit effluent glycol récupéré à l'issue de l'étape b) est avantageusement envoyé à l'étape g) de traitement.

Tout ou partie dudit effluent glycol récupéré à l'issue de l'étape b) peut être pré-purifié dans une section de pré-purification des diols comprise dans l'étape b) pour éliminer une partie des impuretés entrainées avec ledit effluent glycol comme par exemple des colorants, pigments ou autres particules solides. La section de pré-purification alimentée par tout ou partie dudit effluent glycol peut comprendre, de manière non exhaustive, une adsorption sur solide (par exemple sur charbon actif) et un système de filtration. Au moins une fraction dudit effluent glycol pré-purifié peut être directement recyclée vers l'étape a) de dépolymérisation et/ou vers l'étape c) de préparation d'une charge d'estérification.

L'effluent diester obtenu à l'issue de l'étape b) de séparation, avantageusement sous forme liquide, comprend avantageusement plus de 10% poids, de préférence plus de 25% poids, de manière préférée plus de 50% poids de monomères diester et oligomères d'ester.

La section de séparation de l'étape b) peut éventuellement comprendre avantageusement un ou plusieurs systèmes d'évaporation à film tombant ou à film raclé, en série ou en parallèle, opéré(s) à une température inférieure ou égale à 200°C, de manière préférée inférieure ou égale à 180 °C, et à une pression inférieure ou égale à 0,001 MPa, de préférence inférieure ou égale à 0,0005 MPa, pour réduire encore la quantité de diols éventuellement restant dans l'effluent diester tout en minimisant la polymérisation du monomère diester dans ledit effluent diester.

### Etape c) de purification

Ledit effluent diester obtenu à l'issue de l'étape b) de séparation alimente ensuite une étape c) de purification, comprenant au moins une section de séparation puis une section de décoloration.

Avantageusement, l'étape de purification de l'effluent diester obtenu à l'issue de l'étape b) de séparation permet de séparer au moins un effluent diester purifié, de tout ou partie des composés suivants issus de l'étape a) de dépolymérisation : oligomères d'esters, polyester éventuellement non converti, impuretés éventuellement présentes dans la charge polyester à recycler telles que d'autres polymères, des pigments, des colorants, des catalyseurs de polymérisation ou tout autre composé inorganique composant ladite charge polyester à recycler ou formé lors de l'étape a) de dépolymérisation, et le cas échéant des composés diols non encore séparés, tout en minimisant la perte en monomère diester.

Ladite étape de purification permet ainsi de récupérer un effluent diester purifié avec un rendement de monomère diester dans ledit effluent diester purifié supérieur ou égal à 50% poids, de préférence supérieur ou égal à 70% poids, de manière préférée supérieur ou égal à 80% poids. L'expression « rendement de monomère diester dans ledit effluent diester » désigne la quantité de monomère diester dans ledit effluent diester purifié rapportée à la quantité totale de monomère diester introduite dans la section de purification de l'étape b).

De préférence, l'étape de purification met en oeuvre une ou plusieurs opérations de purification, comme la filtration, l'évaporation, la distillation, l'adsorption sur une masse de captation.

L'étape c) de purification comprend une ou plusieurs sections de séparation de l'effluent diester obtenu à l'issue de l'étape b), avec pour objectif de séparer le monomère diester, qui est vaporisé, des impuretés lourdes, en particulier des oligomères et du polyester éventuellement non converti à l'étape a) qui restent liquides et captent donc les impuretés solides, notamment les pigments, du polymère non converti, des autres polymères éventuellement présents et des catalyseurs de polymérisation, tout en minimisant la perte en monomère diester notamment par re-polymérisation. Quelques oligomères d'ester peuvent être entrainés avec le monomère diester.

Ladite (ou lesdites) section(s) de séparation de l'étape c) est(sont) avantageusement alimentée(s) par ledit effluent diester obtenu à l'issue de l'étape b) et opérée(s) à une température inférieure ou égale à 250°C, de manière préférée inférieure ou égale à 230 °C, et de manière très préférée inférieure ou égale à 200°C, à une pression inférieure ou égale à 0,001 MPa, de préférence inférieure ou égale à 0,0001 MPa, de manière préférée inférieure ou égale à 0,00005 MPa, et avec un temps de séjour liquide par section inférieur ou égale à 10 min, de préférence inférieur ou égale à 5 min, de manière préférée inférieur ou égale à 1 min. Le temps de séjour liquide par section est défini, selon la présente description, par le rapport du volume liquide dans ladite section sur le débit volumique du flux le plus chaud sortant de ladite section. A l'issue de ladite (ou lesdites) section(s) de séparation de l'étape c) de purification, au moins un effluent liquide concentré en impuretés lourdes et un effluent diester pré-purifié, de préférence pauvre en impuretés, sont obtenus.

La séparation dudit effluent diester pré-purifié et dudit effluent liquide concentré en impuretés lourdes est avantageusement mise en oeuvre dans un système d'évaporation à film tombant ou à film raclé, par distillation à court trajet à film tombant ou à film raclé, ou par une succession de plusieurs évaporations et/ou distillations court trajet à film tombant ou film raclé, à une température inférieure ou égale à 250°C, de manière préférée inférieure ou égale à 230°C, de manière très préférée inférieure ou égale à 200°C, et à une pression inférieure ou égale à 0,001 MPa, de préférence inférieure ou égale à 0,0001 MPa, de manière préférée inférieure ou égale à 0,00005 MPa. La très faible pression opératoire est nécessaire pour pouvoir opérer ladite séparation à une température inférieure ou égale à 250°C, de préférence inférieure ou égale à 230°C tout en permettant la vaporisation du monomère diester.

Un inhibiteur de polymérisation peut être avantageusement mélangé à l'effluent diester obtenu à l'issue de l'étape b), avant d'alimenter ladite étape c) de purification. Un fluxant peut également être avantageusement mélangé à l'effluent diester issue de l'étape b) avant d'alimenter ladite étape c), de manière à faciliter l'élimination des impuretés lourdes, notamment des pigments, en fond du système d'évaporation ou de distillation court trajet. Lorsqu'il est introduit, le fluxant doit avoir une température d'ébullition très supérieur au monomère diester de l'effluent diester dans les conditions d'opération de l'étape c). Il peut s'agir par exemple de polyéthylène glycol, ou d'oligomères du PET.

L'effluent liquide concentré en impuretés lourdes concentre avantageusement les oligomères, le PET non converti et les impuretés lourdes, notamment les pigments, des autres polymères éventuellement présents et des catalyseurs de polymérisation. Les conditions opératoires de la séparation dans la(ou les) sections de séparation de ladite étape c) sont ajustées de telle manière que la perte en monomères diester par re-polymérisation est minimisée. Quelques oligomères peuvent être entrainés avec le monomère diester dans l'effluent diester pré-purifié, notamment sous forme gazeuse.

Au moins une fraction dudit effluent liquide concentré en impuretés lourdes peut avantageusement être recyclée vers l'étape a) de dépolymérisation pour augmenter le rendement en monomères diester du procédé de dépolymérisation. Avant toute opération de recyclage, ledit effluent liquide concentré en impuretés lourdes subit avantageusement au moins une étape de purification, de manière préférée une étape de filtration de manière à réduire la quantité de pigments et/ou autres impuretés solides éventuellement présentes. Tout ou partie dudit effluent liquide concentré en impuretés lourdes peut également avantageusement être purgé du procédé et envoyé vers un système d'incinération ou vers un système de récupération des pigments.

L'effluent liquide concentré en impuretés lourdes peut avantageusement être mélangé avec au moins une fraction de l'effluent glycol obtenu à l'issue de l'étape b) de séparation et/ou au moins une fraction du flux diol purifié obtenu à l'issu de l'étape g), de manière à diminuer la viscosité dudit effluent liquide concentré en impuretés lourdes et faciliter son transport vers l'étape a) de dépolymérisation, et éventuellement son traitement dans une étape optionnelle de filtration.

Eventuellement, l'effluent diester pré-purifié peut avantageusement être mélangé avec au moins une fraction de l'effluent glycol issu de l'étape b) de séparation et/ou avec au moins une fraction du flux diol purifié issu de l'étape g).

L'étape c) de purification comprend une section de décoloration de l'effluent diester pré-purifié, avantageusement opérée à une température entre 100 et 250°C, de préférence entre 110 et 200°C, et de manière préférée entre 120 et 180°C, et à une pression entre 0,1 et 1,0 MPa, de préférence entre 0,2 et 0,8 MPa, et de manière préférée entre 0,3 et 0,5 MPa, en présence d'un adsorbant, pour produire un effluent diester purifié. Ledit adsorbant peut être tout adsorbant connu de l'Homme du métier apte à capter les colorants, telles que du charbon actif, des argiles, de préférence un charbon actif.

L'effluent diester purifié obtenu à l'issue de l'étape c) comprend au moins un monomère diester, notamment au moins du bis(2-hydroxyéthyl) téréphtalate (BHET). Il peut éventuellement comprendre au moins un diol (ou glycol), de préférence correspondant au motif diol contenu dans ledit monomère diester dudit effluent diester purifié. Avantageusement, l'effluent diester purifié obtenu à l'issue de l'étape c) comprend au moins 10% poids de monomère diester, de préférence au moins 20% poids. Il contient de préférence moins de 1% poids, de préférence moins de 0,1% poids, des pigments introduits dans le procédé avec la charge polyester à recycler et moins de 10% poids, de préférence moins de 1% poids, des colorants introduits dans le procédé avec la charge polyester à recycler. De préférence, l'effluent diester purifié obtenu à l'issue de l'étape c) comprend moins de 10% poids, préférentiellement moins de 1% poids, de manière préférée moins de 0,1% poids d'impuretés plus lourdes que ledit monomère diester autres éventuellement que des oligomères dudit diester. Très avantageusement, l'effluent diester purifié obtenu à l'issue de l'étape c) est exempt d'impuretés colorés ou d'impuretés inorganiques tels que des pigments, des colorants, des catalyseurs de dépolymérisation et des ions. De préférence, le monomère diester purifié comprend les molécules de monomère diester et éventuellement des oligomères dudit diester avec une degré de polymérisation compris entre 2 et 5. Selon au moins du bis(2-hydroxyéthyl) téréphtalate (BHET), le terme « exempt » signifie que la teneur du composé considéré dans l'effluent diester purifié est inférieure à la limite de détermination de la méthode d'analyse utilisée pour déterminer ladite teneur.

Avantageusement, l'effluent diester purifié est obtenu à l'issue de l'étape c) sous forme liquide. Au moins une fraction dudit effluent diester purifié obtenu à l'issue de l'étape c) sous forme liquide alimente l'étape d) du procédé. Au moins une fraction dudit effluent diester purifié peut également être recyclée vers l'étape a) du procédé de production d'un polyester téréphtalate.

### Etape d) de préparation de la charge de polymérisation

Le procédé de production d'un polyester téréphtalate comprend une étape d) de préparation d'une charge de polymérisation. Ladite étape d) comprend au moins une section de mélange alimentée en au moins une charge téréphtalique et au moins une fraction dudit effluent diester purifié obtenu liquide à l'issue de l'étape c). Ladite charge téréphtalique est une charge acide téréphtalique, qui comprend l'acide téréphtalique comme composé à motif téréphtalate, ou une charge diméthyle téréphtalate, qui comprend le diméthyle téréphtalate comme composé à motif téréphtalate.

Dans le mode de réalisation particulier dans lequel la charge téréphtalique est une charge acide téréphtalique comprenant l'acide téréphtalique, la charge de polymérisation qui est obtenue à l'issue de l'étape d), est un mélange diphasique homogène, comprenant au moins de l'acide téréphtalique, un monomère diester et éventuellement un diol (ou glycol). Par « diphasique », on entend avantageusement une suspension d'une phase solide dans une phase liquide ou pâteuse. Par « homogène », il faut entendre que la phase solide, en suspension dans la phase liquide ou pâteuse, est répartie dans l'ensemble de la phase liquide ou pâteuse de façon homogène. Plus particulièrement, la charge de polymérisation dans ce mode de réalisation est un mélange de particules solides d'acide téréphtalique, de diamètre typiquement compris entre 1 et 1000 µm, notamment entre 80 et 300 µm, réparties de façon homogène dans une phase liquide ou pâteuse comprenant le monomère diester.

Dans un autre mode de réalisation particulier dans lequel la charge téréphtalique est une charge diméthyle téréphtalate comprenant le diméthyle téréphtalate, la charge de polymérisation, comprenant au moins le diméthyle téréphtalate, un monomère diester et éventuellement une charge diol (ou glycol), est avantageusement un mélange monophasique. Avantageusement, les quantités en ladite charge téréphtalique, et en effluent diester purifié, introduites dans ladite section de mélange, sont ajustées de sorte que le rapport du nombre total de moles de motifs diol de formule -[C₍ₙ₊₁₎H₍₂ₙ₊₂₎O₂]-, n étant un nombre entier supérieur ou égal à 1, de préférence compris entre 1 et 5, préférentiellement entre 1 et 3, introduits dans ladite section de mélange, par rapport au nombre total de moles de motifs téréphtalate de formule -[CO-(C₆H₄)-CO]-, introduits dans ladite section de mélange, est compris entre 1,0 et 2,0, de préférence entre 1,0 et 1,5, préférentiellement entre 1,0 et 1,3.

Selon un mode de réalisation, ladite section de mélange de ladite étape d) du procédé est en outre alimentée en une charge monomère diol, de préférence comprenant un monomère diol de formule chimique HO-C₍ₙ₊₁₎H₍₂ₙ₊₂₎-OH, n étant un nombre entier supérieur ou égal à 1, de préférence entre compris 1 et 5, de manière préférée compris entre 1 et 3. De préférence, ladite charge monomère diol comprend au moins 90% mol., préférentiellement au moins 95% mol., de manière très préférée 98% mol., d'un monomère diol entant dans la composition du motif unitaire du polyester téréphtalate visé. De manière préférée, la charge monomère diol comprend de l'éthylène glycol, de préférence à une teneur supérieure ou égale à 90% mol., au moins 95% mol., préférentiellement au moins 98% mol.. Le diol de ladite charge monomère diol peut être de structure chimique différente ou de même structure chimique, de préférence de même structure chimique, que celle du motif diol entrant dans la composition de la charge polyester à recycler de l'étape a) de dépolymérisation. De préférence, ladite charge en monomère diol peut être, au moins en partie, une fraction du flux diol purifié obtenu à l'étape g) et/ou au moins en partie une fraction de l'effluent glycol obtenu à l'issu de l'étape b) .

Lorsqu'une charge monomère diol est incorporée dans la section de mélange de l'étape d), la quantité en ladite charge diol introduite dans la section de mélange de l'étape d) est ajustée de sorte que le rapport du nombre de motifs diol par rapport au nombre de motifs téréphtalate dans le mélange de l'étape d), comme défini ci-dessus, est compris entre 1,0 et 2,0, de préférence entre 1,0 et 1,5, préférentiellement entre 1,0 et 1,3.

Avantageusement, la quantité de molécules de monomère diester de l'effluent diester purifié introduit dans la section de mélange de l'étape d) représente au moins 5% poids par rapport au poids d'acide téréphtalique (PTA), ou diméthyle téréphtalate (DMT), de préférence au moins 15 % poids.

Dans la mesure où une molécule de monomère diester comprend deux motifs diol et un motif téréphtalate, une molécule d'acide téréphtalique ou de diméthyle téréphtalate comprend un motif téréphtalate et une molécule de diol comprend un motif diol, l'incorporation d'une mole de monomères diester, par exemple une mole de bis(2-hydroxyéthyl) téréphtalate (BHET), en mélange avec les charges monomères acide téréphtalique ou diméthyle téréphtalate et diol, tel que l'éthylène glycol, permet de substituer une partie de ladite charge téréphtalique et tout ou partie de ladite charge diol.

Avantageusement, le mélange de ladite charge téréphtalique, d'au moins un fraction dudit effluent diester purifié et éventuellement de ladite charge monomère diol est réalisé dans tout équipement connu de l'Homme du Métier et permettant un mélange efficace. Dans le mode de réalisation particulier dans lequel la charge de polymérisation comprend l'acide téréphtalique, l'étape d) permet d'obtenir une répartition homogène des solides en suspension dans le mélange liquide ou la pâte. De préférence, le mélange de l'étape d) est mis en oeuvre dans un mélangeur avec une agitation mécanique ou agité par recirculation d'un liquide ou dudit mélange, par exemple à l'aide d'une pompe mettant en circulation le mélange dans une boucle.

Ladite charge téréphtalique, la fraction dudit effluent diester purifié et éventuellement ladite charge monomère diol sont introduites ensemble ou séparément dans le mélangeur.

Avantageusement, ladite section de mélange dans l'étape d) est opérée à une température comprise entre 25 et 250°C, de préférence entre 60 et 200°C, de manière préférée entre 100 et 150°C, et à une pression supérieure ou égale à 0,1 MPa. La pression de ladite section de mélange est très avantageusement inférieure ou égale à 5 MPa.

Un ou plusieurs catalyseurs de polymérisation peu(ven)t, en outre, être incorporé(s) dans le mélange de l'étape d).

D'autres composés monomères (ou co-monomères) peuvent également être avantageusement introduits dans le mélange et se retrouver dans la charge d'estérification. De manière non exhaustive, lesdits autres composés monomères peuvent être des acides dicarboxyliques tels que par exemple l'acide iso-phtalique et des diols tels que par exemple le 1,4-dihydroxy-méthylcyclohexane et le diéthylène glycol.

Selon un des modes particuliers dans lequel une charge acide téréphtalique est utilisée, l'incorporation dans la charge de polymérisation d'un monomère diester, par exemple des monomères BHET, permet de substituer une partie de l'acide téréphtalique, qui est un composé sous forme de poudre de particules solides, dans le mélange diphasique des monomères pour la production de polyester. Cette substitution d'une partie de l'acide téréphtalique et de tout ou partie du diol par des monomères diester permet, à taux de solide du mélange diphasique identique par rapport au celui des procédés classiques de production de polyester, de diminuer de façon conséquente la quantité de diol, par exemple d'éthylène glycol, introduit en excès dans le mélange, induisant une réduction des coûts notamment de matières premières mais aussi une réduction importante des consommations énergétiques du procédé de production de polyester, du fait de la diminution de la quantité de diol à récupérer au cours de la polymérisation, à traiter et à recycler.

### Etape e) de condensation

Le procédé de production d'un polyester téréphtalate comprend une étape d) de condensation de la charge de polymérisation obtenue à l'issue de l'étape d), pour produire au moins un effluent réactionnel de condensation, un effluent diol et un effluent aqueux ou un effluent méthanol. Ladite étape e) de condensation comprend au moins une section réactionnelle et au moins une section de séparation.

Dans le mode de réalisation particulier dans lequel la charge téréphtalique est une charge acide téréphtalique, la réaction mise en oeuvre à l'étape e) comprend avantageusement une réaction d'estérification qui consiste en une réaction de condensation d'au moins les groupes hydroxyles (-OH) des monomères diester de la fraction de l'effluent diester purifié, produit à l'étape c) et incorporé au moins en partie à la charge de polymérisation à l'étape d), et des monomères diol éventuellement présents dans la charge de polymérisation, avec au moins les groupes carboxyles (-COOH) de l'acide téréphtalique de la charge acide téréphtalique incorporée à la charge de polymérisation à l'étape d). Cette réaction d'estérification produit des molécules de monomère diester, par exemple du bis(2-hydroxyethyl) téréphtalate (BHET), et des oligomères de diester comprenant avantageusement 2 à 5 motifs téréphtalate. Elle libère également de l'eau. La réaction mise en oeuvre à l'étape e) comprend également avantageusement des réactions de transestérification consistant en la réaction de condensation de molécules de monomère diester entre elles, libérant ainsi des molécules de diol.

Dans l'autre mode de réalisation particulier dans lequel la charge téréphtalique est une charge diméthyle téréphtalate, la réaction mise en oeuvre à l'étape e) comprend avantageusement une réaction de transestérification qui consiste en une réaction de condensation d'au moins les groupes hydroxyles (-OH) des monomères diester de la fraction de l'effluent diester purifié, produit à l'étape c) et incorporé au moins en partie à la charge de polymérisation à l'étape d), et des monomères diol éventuellement présents dans la charge de polymérisation, avec au moins les groupes carboxylate (-COO-) de diméthyle téréphtalate, de la charge diméthyle téréphtalate incorporée à la charge de polymérisation à l'étape d). Cette réaction de transestérification produit des molécules de monomère diester, par exemple du bis(2-hydroxyethyl) téréphtalate (BHET), et des oligomères de diester comprenant avantageusement 2 à 5 motifs téréphtalate. Elle libère également du méthanol. La réaction mise en oeuvre à l'étape e) comprend également avantageusement des réactions de transestérification consistant en la réaction de condensation de molécules de monomère diester entre elles, libérant ainsi des molécules de diol.

Avantageusement, ladite section réactionnelle est opérée dans un ou plusieurs réacteurs en série ou parallèle, à une température entre 150 et 400°C, de préférence entre 200 et 300°C, à une pression entre 0,05 et 1 MPa, de préférence entre 0,1 et 0,3 MPa, et avec un temps de séjour entre 0,5 et 10 h, de préférence entre 1 et 5 h. Selon la présente description, le temps de séjour dans ladite étape d) d'estérification est défini comme le rapport du volume réactionnel d'un réacteur de ladite section réactionnelle sur le débit volumique du flux liquide sortant dudit réacteur.

Avantageusement, un catalyseur de polymérisation connu de l'Homme du métier, peut être éventuellement introduit, éventuellement en mélange avec un flux diol, dans la section réactionnelle de l'étape e) de condensation, en particulier dans le cas d'une réaction d'estérification à partir d'une charge de polymérisation. Les catalyseurs de polymérisation sont de manière non exhaustive des catalyseurs à base d'antimoine, de titane, de germanium ou d'aluminium, de l'acétate de zinc, de calcium ou de manganèse. Dans le mode de réalisation particulier dans lequel la charge de polymérisation comprend la charge diméthyle téréphtalate, un catalyseur de transestérification connu de l'Homme du métier est éventuellement ajouté dans la section réactionnelle de l'étape e), avantageusement en mélange avec un flux diol.

Les réactions de condensation sont avantageusement mises en oeuvre dans un ou plusieurs réacteurs agités, dans un ou plusieurs réacteurs tubulaires ou dans une association de réacteurs agités et tubulaires.

Avantageusement, la section réactionnelle comprend également au moins un soutirage d'un effluent soutiré, riche en eau ou en méthanol, et en diol. L'eau ou le méthanol et le diol compris dans ledit effluent soutiré sont séparés dans la section de séparation de l'étape e). Cette dernière est alimentée en ledit effluent soutiré, riche en eau ou méthanol et en diol, et produit avantageusement un effluent aqueux, ou méthanol, et un effluent diol. Avantageusement, l'eau, ou le méthanol, est séparé(e) par différence de volatilité, par exemple par distillation, ou par adsorption à partir dudit effluent soutiré et contenant au moins une partie du diol et de l'eau ou du méthanol libérés présents dans le milieu réactionnel.

Avantageusement, au moins une fraction dudit effluent diol obtenu à l'étape e) du procédé peut éventuellement être envoyée vers l'étape g) de traitement des diols ou directement renvoyée à la section réactionnelle de ladite étape e). De préférence, ledit effluent diol obtenu à l'issue de la section de séparation de l'étape e) est renvoyé directement à la section réactionnelle de ladite étape e).

Ledit effluent réactionnel de condensation obtenu à l'issue de ladite étape e), en particulier à la sortie de la section réactionnelle de l'étape e), comprend des monomères diesters et des oligomères d'ester. De préférence, les monomères diesters dans ledit effluent réactionnel de condensation sont de même nature que les monomères diester de l'effluent diester purifié, liquide, obtenu à l'étape c) et incorporé au moins en partie dans le mélange de l'étape d). De préférence, les oligomères d'ester dans ledit effluent réactionnel de condensation se composent avantageusement de motifs élémentaires correspondant aux motifs élémentaires de répétition du polyester téréphtalate visé par le procédé tel que décrit dans la présente description.

L'incorporation d'au moins une fraction de l'effluent diester purifié aux charges monomères de la polymérisation permet de substituer au moins une partie de la charge téréphtalique, et tout ou partie de la charge diol, permettant ainsi de réduire la quantité d'eau ou méthanol libéré et donc de limiter la quantité d'effluent aqueux ou méthanol, soutiré du milieu réactionnel et à traiter. La consommation énergétique s'en trouve avantageusement diminuée.

### Etape f) de polycondensation

Le procédé de production d'un polyester téréphtalate comprend une étape f) de polycondensation de l'effluent réactionnel de condensation obtenu à l'étape e), pour obtenir au moins ledit polyester téréphtalate et un effluent diol. Ledit effluent diol comprend au moins un monomère diol correspond avantageusement au motif diol de formule -[C₍ₙ₊₁₎H₍₂ₙ₊₂₎O₂]-, n étant un nombre entier supérieur ou égal à 1, de préférence compris entre 1 et 5, préférentiellement entre 1 et 3, du monomère diester de l'effluent diester purifié qui alimente au moins en partie la section de mélange de l'étape d) du procédé.

L'étape f) de polycondensation consiste à réaliser une réaction de condensation entre les monomères diester et oligomères d'esters de l'effluent réactionnel de condensation obtenu à l'étape e) pour obtenir un polyester avec un degré de polymérisation donné et les propriétés physico-chimiques désirés (par exemple : indice de viscosité, cristallinité, couleur, propriétés mécaniques,...). Ladite réaction de condensation libère des composés diols, éventuellement de l'eau ou méthanol et des co-produits, qu'il convient d'éliminer.

L'étape f) de polycondensation comprend au moins une section réactionnelle comprenant au moins un réacteur dans lequel la réaction de polycondensation est mise en oeuvre et au moins un soutirage d'un effluent diol comprenant au moins un monomère diol, correspondant avantageusement au motif diol de formule -[C₍ₙ₊₁₎H₍₂ₙ₊₂₎O₂]-, n étant un nombre entier supérieur ou égal à 1, de préférence compris entre 1 et 5, préférentiellement entre 1 et 3, du monomère diester de l'effluent diester purifié.

Avantageusement, ladite section réactionnelle de l'étape f) est opérée, dans un ou plusieurs réacteurs, fonctionnant en série ou en parallèle, à une température 200 et 400°C, de préférence entre 250 et 300°C, à une pression entre 0,0001 et 0,1 MPa, de préférence entre 0,0004 et 0,01 MPa, avec un temps de séjour entre 0,1 et 5 h, de préférence entre 0,5 et 3 h. Selon la présente description, le temps de séjour dans ladite étape f) de polycondensation est défini comme le rapport du volume réactionnel d'un réacteur de ladite section réactionnelle sur le débit volumique du flux liquide sortant dudit réacteur La réaction de polycondensation de l'étape f) peut être réalisée en deux étapes réactionnelles successives : une étape de condensation en phase fondue et suivie d'une étape de post-condensation en phase solide.

Avantageusement, des additifs et catalyseurs de polymérisation peuvent être introduits à l'étape f) de polycondensation. De manière non exhaustive, les additifs peuvent comprendre des agents d'inhibition des réactions secondaires d'éthérification comme par exemple des amines (n-butylamine, diisopropylamine ou triethylamine), de l'hydroxyde de sodium ou des hydroxydes organiques ou du carbonate de lithium, des agents stabilisants tel que des phosphites ou des phosphates, et des composés de type polyamides pour réduire la quantité de produit de dégradation comme l'acétaldéhyde. Les catalyseurs de polymérisation couramment utilisés, sont comme par exemple, des catalyseurs à base d'antimoine, de titane, de germanium ou d'aluminium, de l'acétate de zinc, de calcium ou de manganèse.

Avantageusement, le soutirage dudit effluent diol est mis en oeuvre à l'aide d'un ou plusieurs système(s) de soutirage, avantageusement connecté au(x) réacteur(s) de la section réactionnelle de ladite étape f), et permet de séparer le monomère diol libéré lors de la réaction de polycondensation et éventuellement l'eau, ou méthanol, et d'autres co-produits éventuellement libérés au cours de la réaction de condensation. De préférence, l'effluent diol, soutiré du(des) réacteurs de l'étape f), est soutiré sous forme gazeuse et est ensuite avantageusement refroidi à une température entre 0 et 100°C et condensé pour obtenir un effluent diol liquide.

De manière préférée, au moins une fraction de l'effluent diol liquide est envoyé à l'étape g). Au moins une fraction dudit effluent diol liquide peut également être recyclée directement à l'étape d) de préparation d'une charge de polymérisation. Dans un mode de réalisation très particulier, ledit effluent diol liquide peut être tout ou partie recyclé directement vers l'étape e) de condensation.

### Etape g) de traitement des diols

Le procédé de production d'un polyester téréphtalate comprend une étape g) de traitement des diols, comprenant une section de récupération alimentée au moins par tout ou partie de l'effluent glycol issu de l'étape b), et tout ou partie de l'effluent diol issu de l'étape f), pour obtenir un flux diol à traiter, et une section de purification dudit flux diol à traiter pour obtenir un flux diol purifié.

Ladite section de récupération de l'étape g) peut en outre être éventuellement alimentée par au moins une fraction de l'effluent diol issue de l'étape e) de condensation et/ou un appoint externe de diol.

Avantageusement, la section de récupération peut comprendre une ou plusieurs opérations de filtration.

Ledit flux diol à traiter obtenu à l'issue de la section de récupération de l'étape g) est envoyé vers la section de purification de l'étape g) pour obtenir un flux diol purifié.

Ladite section de purification comprend au moins un système de séparation mettant en oeuvre toute méthode de séparation physique, physico-chimique ou chimique connue de l'Homme du métier, comme par exemple la séparation gaz-liquide, la distillation, l'adsorption. De préférence, la purification dudit flux diol à traiter met en oeuvre au moins une colonne de distillation, de préférence une série de colonnes de distillation, opérée(s) à une température entre 50 et 250°C, de préférence entre 70 et 220°C et à une pression entre 0,001 et 0,2 MPa, de préférence entre 0,01 et 0,1 MPa. De manière préférée, ladite section de purification comprend une phase de séparation des impuretés plus légères que le monomère diol compris dans le flux diol à traiter et une phase de séparation des impuretés plus lourdes que le monomère diol compris dans le flux diol à traiter, de préférence dans une série de colonnes de distillation.

Avantageusement, ladite étape g) peut également comprendre une section d'élimination des composés organiques volatiles par combustion thermique ou catalytique desdits composés pour éviter leur rejet dans l'environnement. De manière non exhaustive, ladite section de traitement des impuretés comprend une filtration si présence de particules solides et un système de combustion catalytique ou non.

Ledit flux diol purifié obtenu à l'issue de l'étape g) peut alors être envoyé, en tout ou partie, vers au moins les étapes a) et d).

Le procédé de production d'un polyester téréphtalate tel que décrit permet ainsi d'obtenir un polyester téréphtalate, ayant avantageusement le degré de polymérisation visé et les propriétés physico-chimiques désirées, à partir de matière polyester à recycler issue des filières de collecte et de tri, tout en limitant la consommation de matière première « fraîche » et la consommation énergétique globale. La réduction de la consommation énergétique est notamment permise grâce à un système de recyclage optimisé du(des) diol(s), le diol libéré notamment au cours de la polymérisation et le diol éventuellement éventuellement introduit dans le mélange de monomères pour la polymérisation (c'est-à-dire dans la charge de polymérisation) et qui n'a pas été converti.

Le procédé, à l'issue des étapes a),b) et c), permet également, très avantageusement, de produire un flux intermédiaire comprenant un diester de l'acide téréphtalique (comme par exemple le BHET) et présentant une pureté élevée compatible avec les spécifications requises par les procédés de polymérisation pour obtenir un polyester téréphtalate, ledit flux intermédiaire diester liquide pouvant être injecté directement dans les étapes dites de polymérisation, notamment directement incorporé à un mélange de charges monomères alimentant les section de polymérisation, de préférence en substitution d'au moins une partie desdites charges monomères. L'injection directe dudit flux intermédiaire diester permet d'avoir une consommation énergétique plus faible que la somme d'un procédé de dépolymérisation et d'un procédé de polymérisation. De préférence, le diester de l'acide téréphtalique est le bis(2-hydroéthyl) téréphtalate (BHET) issu de la glycolyse du PET par l'éthylène glycol.

Le procédé de production d'un polyester téréphtalate tel que décrit permet de répondre à une demande écologique, puisqu'elle permet le recyclage de matière plastique, jusqu'à présent difficile à recycler comme le PET opaque.

Les figures et exemples suivants illustrent l'invention sans en limiter la portée.

### Liste des figures

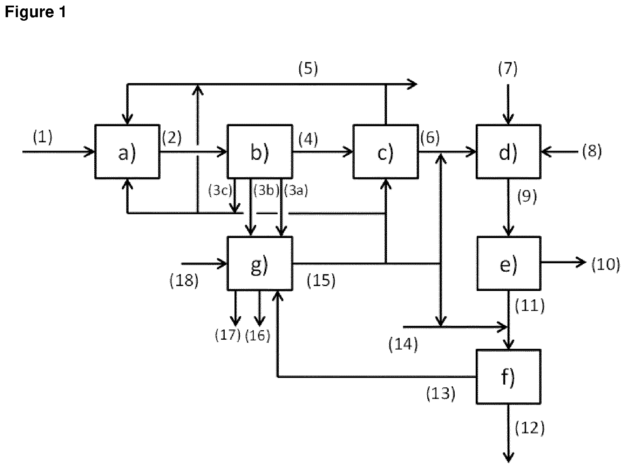

La Figure 1 schématise une configuration particulière d'un procédé de production de polyéthylène téréphtalate (PET) selon l'invention, intégrant un schéma de dépolymérisation, de préférence par glycolyse, d'une charge PET à recycler et un schéma de polymérisation d'un mélange comprenant le flux de BHET purifié intermédiaire obtenu du procédé de dépolymérisation, de l'acide téréphtalique et du mono-éthylène glycol diol (ou éthylène glycol, MEG).

Le procédé de glycolyse de la Figure 1 comprend la succession des étapes a) de dépolymérisation puis b) de séparation puis c) de purification.

L'étape a) de dépolymérisation alimenté par au moins ladite charge PET à recycler (1) et une charge de MEG (3c). Avantageusement, ladite charge PET à recycler est préalablement chauffée et mise en pression aux conditions opératoires de ladite étape a). Avantageusement, au moins 80% poids de la charge est introduite dans l'étape a) sous forme liquide, très avantageusement au moins 90% poids, préférentiellement au moins 95% poids. La température de la charge PET à recycler est avantageusement comprise entre 225 et 275°C.

Le temps nécessaire à l'introduction et la température associée sont ajustés de manière à minimiser la dégradation thermique du polyester.

Avantageusement, ladite étape a) comprend une section de convoyage à vis, dite section d'extrusion, alimentée par ladite charge PET à recycler. Le temps de séjour dans ladite section d'extrusion, défini comme le volume de ladite section divisé par le débit volumique de charge est avantageusement inférieur à 15 min, de préférence inférieur à 10 min, et de manière préférée inférieure à 2 min. Ladite section d'extrusion est avantageusement connectée à un système d'extraction sous vide de manière à éliminer des impuretés telles que des gaz dissous, des composés organiques légers et/ou de l'humidité présents dans la charge PET à recycler. Ladite section d'extrusion peut également avantageusement comprendre un système de filtration pour éliminer des particules solides de taille supérieure à 40 µm, de préférence de taille comprise entre 3 et 40 µm, telles que des particules de sable.

La charge PET à recycler est avantageusement mise en contact avec au moins une fraction de l'effluent MEG (3c) issu de l'étape b), avantageusement au sein de ladite section d'extrusion, appelée aussi section d'extrusion réactive. Le flux MEG (3c) peut avantageusement être surchauffé préalablement à son alimentation dans l'étape a) afin de faciliter la mise en température de la charge PET à recycler. Le nombre de moles de MEG provenant de l'étape b) par moles de diester dans ladite charge PET à recycler est avantageusement inférieur ou égal à 1,0 et de manière préférée inférieur ou égal à 0,5.

Ladite étape a) peut également avantageusement être alimentée en une fraction de l'effluent (5) issu de l'étape c), ladite fraction comprenant un mélange d'oligomères et de polyester non convertis et ayant préférentiellement été purifiée dans une étape de filtration.

L'étape a) de dépolymérisation comprend une section réactionnelle opérée, dans un ou plusieurs réacteurs, à une température comprise entre 150 et 400°C, de préférence entre 180 et 300°C, de manière préférée entre 200°C et 280°C, en phase liquide, avec de 1 à 20 moles de MEG par mole de diester dans ladite charge PET à recycler, de préférence de 3 à 15 moles par mole, et de manière préférée de 5 à 10 moles par mole, un temps de séjour compris entre 0,1 et 6 h de préférence entre 0,5 et 4 h, et une pression d'opération est d'au moins 0,1 0,4 MPa, préférentiellement au moins 0,4 MPa. De préférence, ladite étape de dépolymérisation est réalisée sans adjonction de catalyseur.

La réaction de glycolyse permet de convertir la charge PET à recycler en monomère Bis(2-Hydroxyethyl) téréphtalate (BHET) et oligomères de BHET. La conversion de la charge PET à recycler dans ladite étape de dépolymérisation est supérieure à 50 %, de préférence supérieure à 70 %, de manière préférée supérieure à 85%. Le rendement molaire en BHET est supérieur à 50 %, de préférence supérieur à 70%, de manière préférée supérieur à 85%. Le rendement molaire en BHET correspond au débit molaire de BHET en sortie de ladite étape a) sur le débit de moles de motifs diester compris dans la charge PET à recycler (1).

Une boucle interne de recirculation est également avantageusement mise en oeuvre dans l'étape a). Elle comprend le soutirage d'une fraction du système réactionnel (c'est-à-dire l'ensemble des constituants et phases présents au sein de ladite section réactionnelle), la filtration de cette fraction, et la réinjection de ladite fraction dans ladite étape a). Cette boucle interne de recirculation permet avantageusement d'éliminer les impuretés solides éventuellement comprises dans le liquide réactionnel.

L'effluent réactionnel (2) issu de l'étape a) de dépolymérisation alimente l'étape b) de séparation du MEG, qui est opérée à une pression inférieure à celle de l'étape a) de manière à vaporiser une fraction de l'effluent réactionnel (2) en au moins un effluent MEG gaz comprenant plus de 50% poids de MEG, de préférence plus de 70% poids, de manière préférée plus de 90% poids, et un effluent liquide riche en monomères BHET (4).

L'étape b) comprend avantageusement une succession de séparation gaz-liquide, avantageusement de 1 à 5, très avantageusement de 3 à 5, séparateurs gaz-liquide successifs, opérées à une température comprise entre 100 et 250°C, de préférence entre 110 et 220°C, de manière préférée entre 120 et 210 °C. L'effluent liquide du séparateur antérieur alimente le séparateur ultérieur. L'effluent liquide issu de la dernière séparation gaz-liquide constitue l'effluent liquide riche en monomères BHET (4). Au moins une fraction des effluents MEG gaz récupérés est condensée en effluents MEG liquides.

La température et la pression du séparateur ultérieur est inférieure à celle du séparateur antérieur de manière à ce que au moins une partie de l'effluent MEG gaz sortant de la séparation antérieure puisse, en se condensant, rebouillir une partie de l'effluent liquide de la séparation ultérieure. Dans cette configuration, l'apport de chaleur pour récupérer le MEG est minimisé.

L'étape b) est opérée de telle sorte que la température des effluents liquides soit maintenue au-dessus de la température de précipitation du monomère BHET, et en dessous d'une valeur haute, dépendant du ratio molaire diol/monomère, au-dessus de laquelle le monomère BHET se re-polymérise de manière significative. La température dans les sections de séparation gaz-liquide de l'étape b) est comprise entre 100 et 250°C, de préférence entre 110 et 220°C, de manière préférée entre 120 et 210 °C. La pression dans les séparateurs gaz-liquide de l'étape b) est ajustée pour permettre l'évaporation du MEG et des impuretés éventuellement présentes dans l'effluent réactionnel (2) à une température minimisant la re-polymérisation et permettant une intégration énergétique optimale. Elle est généralement comprise entre 0,00001 et 0,2 MPa, de préférence entre 0,00004 et 0,15 MPa, de manière préférée entre 0,00004 et 0,1 MPa.

Les effluents MEG gaz et liquides issus desdites séparations gaz-liquide peuvent contenir d'autres composés comme des colorants, des alcools légers, de l'eau, du diéthylène glycol. L'étape b) comprend avantageusement une ou plusieurs sections de fractionnement de tout ou partie desdits effluents MEG gaz et liquides en au moins un effluent riche en impuretés légères (3a), un effluent riche en impuretés lourdes (3b) et un effluent MEG (3c), et opérée(s) à une température comprise entre 50 et 250°C, de préférence entre 60 et 210°C, de manière préférée entre 70 et 180°C, à une pression comprise entre 0,00001 et 0,2 MPa, de préférence entre 0,00004 et 0,15 MPa, de manière préférée entre 0,00004 et 0,1 MPa. De préférence, le fractionnement desdits effluent MEG gaz et liquides est mis oeuvre dans des colonnes de distillation, de stripage ou de rectification. Avantageusement, tout ou partie desdits effluents MEG peut être traitée dans une étape de pré-purification, en amont ou aval desdites sections de fractionnement, pour éliminer les colorants par exemple par une adsorption sur solide (par exemple sur charbon actif).

L'effluent MEG 3c) contient avantageusement plus de 99% poids de MEG, de préférence plus de 99,5% poids de MEG. Tout ou partie de l'effluent MEG (3c) est avantageusement recyclé vers l'étape a), avantageusement en mélange avec au moins une fraction de l'effluent MEG purifié issue de l'étape g) de récupération du procédé de polymérisation.

L'effluent riche en impuretés légères (3a) et l'effluent riche en impuretés lourdes (3b) sont avantageusement envoyés à l'étape g) de récupération du procédé de polymérisation.

L'effluent liquide riche en monomères BHET (4) alimente l'étape c) de purification. L'étape c) comprend une ou plusieurs sections de séparation de l'effluent liquide riche en monomères BHET (4) en effluent concentré en impuretés lourdes liquide (5) et un effluent BHET pré-purifié, opérée(s) à une température inférieure à 250°C, de manière préférée inférieure à 230 °C, et de façon très préférée inférieure à 200°C, et une pression inférieure à 0,001 MPa, de préférence inférieure à 0,0001 MPa, de manière préférée inférieure ou égale à 0,00005 MPa, avec un temps de séjour liquide inférieur à 10 min, de préférence inférieur à 5 min, de manière préférée inférieur à 1 min.

L'effluent concentré en impuretés lourdes liquide (5) concentre avantageusement les oligomères, le PET non converti et les impuretés lourdes, notamment les pigments, des autres polymères éventuellement présents et des catalyseurs de polymérisation. Les conditions opératoires de la séparation dans ladite étape c) est ajustées de tel manière que la perte en monomères BHET par re-polymérisation est minimisée. Quelques oligomères peuvent être entrainés avec le monomère dans l'effluent BHET pré-purifié gaz.

La séparation dudit effluent BHET pré-purifié est avantageusement mise en oeuvre dans un système d'évaporation à film tombant ou film raclé ou par distillation à court trajet à film tombant ou à film raclé ou une succession de plusieurs évaporations et/ou distillations court trajet à film tombant ou film raclé. La très faible pression opératoire est nécessaire pour pouvoir opérer ladite séparation à une température inférieure à 250°C, de préférence inférieure à 230°C tout en permettant la vaporisation du monomère.

Une fraction dudit effluent concentré en impuretés lourdes (5) peut avantageusement être recyclé vers l'étape a) de dépolymérisation pour augmenter le rendement en BHET du procédé de dépolymérisation.

Ledit effluent impuretés lourdes (5) subit avantageusement au moins une étape de purification, de manière préférée une étape de filtration préalablement à son recyclage de manière à réduire la quantité de pigments et/ou autres impuretés solides. Tout ou partie dudit effluent impuretés lourdes (5) peut également avantageusement être purgé du procédé et envoyé vers un système d'incinération ou vers un système de récupération des pigments.

Une fraction de l'effluent MEG (3c) ou de l'effluent MEG purifié (15) issu de l'étape g) ou un mélange d'une fraction desdits effluents peut avantageusement être mélangée avec l'effluent concentré en impuretés lourdes (5) issu de l'étape c) de manière à diminuer la viscosité dudit effluent impuretés lourdes et faciliter son transport vers l'étape a), et éventuellement son traitement dans une étape optionnelle de filtration.

L'étape c) comprend une ou plusieurs sections de décoloration de l'effluent BHET pré-purifié, opérée à une température comprise entre 100 et 250°C, de préférence entre 110 et 200°C, et de manière préférée entre 120 et 180°C, et à une pression comprise entre 0,1 et 1,0 MPa, de préférence entre 0,2 et 0,8 MPa, et de manière préférée entre 0,3 et 0,5 MPa en présence d'un adsorbant et produisant un effluent BHET purifié (6). Ledit adsorbant peut être tout adsorbant connu de l'Homme du métier apte à capter les colorants, telles que du charbon actif, des argiles, avantageusement un charbon actif.

L'effluent BHET pré-purifié est avantageusement mélangé avec une fraction de l'effluent MEG 3c) issu de l'étape b) ou avec une fraction de l'effluent MEG purifié issu de l'étape g).

L'effluent BHET purifié (6) obtenu à l'issu de l'étape c) du procédé de dépolymérisation comprend avantageusement plus de 50% molaire de MEG, de préférence plus de 60% molaire, de manière préférée plus de 70 % molaire.

L'effluent BHET purifié (6) alimente avantageusement une étape d) de préparation de charges. Il est mélangé, à l'étape d), avec au moins une charge acide téréphtalique (7), éventuellement avec une partie de l'effluent MEG (15) issu de l'étape g) et éventuellement des co-monomères (8).

L'étape d) de préparation des charges est opérée à une température de préférence supérieure à 80°C, de manière préférée supérieure à 110°C.

Dans le cas où une unité de dépolymérisation est rattachée à une unité déjà existante de polymérisation, la quantité de monomère BHET issu de l'effluent BHET purifié (6) peut représenter avantageusement moins de 30 % molaire de la quantité totale de monomère aromatique comprenant l'acide téréphtalique le BHET dans le mélange préparé à l'étape d) du procédé de polymérisation, de préférence moins de 25 % molaire, de manière préférée moins de 20 % molaire, ce qui permet d'assurer une intégration optimale en minimisant l'investissement sur l'unité de dépolymérisation et les modifications à apporter sur l'unité de polymérisation.

Le procédé de polymérisation comprend également au moins une étape e) d'estérification, une étape f) de polycondensation et une étape g) de récupération et purification des effluents MEG.

Le procédé de polymérisation est de préférence un procédé déjà opérant c'est-à-dire déjà opéré avec des charges monomères PTA et MEG, éventuellement des co-monomères avant l'intégration du procédé de dépolymérisation de PET à recycler. L' intégration du procédé de dépolymérisation dans ladite configuration particulière du procédé permet d'incorporer du PET à recycler dans du PET vierge sans aucun impact sur les propriétés du produit PET final tout en minimisant les impacts sur la configuration du procédé de polymérisation et la consommation énergétique grâce à une intégration optimisée du procédé de dépolymérisation avec les étape d), e) et g) du procédé de polymérisation, ainsi qu'aux performances et à la faible consommation énergétique dudit procédé de dépolymérisation.

L'étape e) d'estérification est alimentée par le mélange (9) issu de l'étape d). La réaction d'estérification comprend une section réactionnelle opérée, dans un ou plusieurs réacteurs, à une température entre 150 et 400°C, de préférence entre 200 et 300°C, à une pression entre 0,05 et 1 MPa, de préférence entre 0,1 et 0,3 Mpa, et un temps de séjour entre 1 et 10 h, de préférence entre 1,5 et 5 h.

L'eau produite par la réaction d'estérification est avantageusement séparée par au moins un système de séparation d'eau compris dans l'étape e), de préférence par distillation, pour produire un flux aqueux (10).

L'effluent réactionnel (11) alimente ensuite l'étape f) de polycondensation. Ladite étape f) est avantageusement alimentée par un catalyseur de polymérisation et des additifs (14) de préférence en mélange avec une fraction de l'effluent MEG purifié (15) issu de l'étape g).

L'étape f) de polycondensation comprend au moins section réactionnelle pour produire un flux polyester (12) et au moins un soutirage permettant de séparer au moins un effluent (13) contenant le MEG, l'eau et autres co-produits libérés au cours des réactions de condensation. Ledit effluent (13) est avantageusement envoyé à l'étape g) de récupération et purification des effluents MEG.

La section réactionnelle de l'étape f) de polycondensation comprend un ou plusieurs réacteurs opérés à une température entre 200 et 400°C, de préférence entre 250 et 300°C, à une pression entre 0,0001 et 0,1 MPa, de préférence entre 0,0004 et 0,01 MPa, avec un temps de séjour entre 0,1 et 5 h, de préférence entre 0,5 et 3 h.

L'étape g) de récupération et purification des effluents MEG récupère avantageusement l'effluent (13) issu de l'étape f) du procédé de polymérisation et les effluents 3a) et 3b) issu de l'étape b) du procédé de dépolymérisation.

Ladite étape g) comprend une ou plusieurs sections de séparation, de préférence une section de séparation des impuretés plus légères que le MEG et une section de séparation des impuretés plus lourdes que le MEG. De préférence, la purification desdits effluents MEG est réalisée par une série de deux colonnes de distillation opérées à une température entre 50 et 250°C, de préférence entre 70 et 220°C et à une pression entre 0,001 et 0,2 MPa, de préférence entre 0,01 et 0,1 MPa.

L'effluent 3a) riche en impuretés légères est avantageusement mélangé à l'effluent (13) puis envoyé à une première colonne de distillation permettant de séparer les impuretés légères (16), en particulier l'eau, d'un effluent MEG qui est ensuite avantageusement envoyé en mélange avec l'effluent 3b) riche en impuretés lourdes dans une seconde colonne de distillation pour récupérer un effluent MEG purifié (15) et un effluent d'impuretés lourdes (17). Les performances du procédé de dépolymérisation minimisant la production d'impuretés légères et lourdes, et le pré-fractionnement réalisé avantageusement à l'étape b) dudit procédé de dépolymérisation permet d'alimenter les colonnes de séparation de l'étape g) du procédé de polymérisation avec un impact mineur sur leur configuration initiale.

### Exemples

### EXEMPLE 1 - Comparatif

Production de PET vierge dans un procédé de polymérisation de PET 5,5 t/h d'acide téréphtalique (PTA) sont introduits dans un ballon mélangeur équipé d'une agitation mécanique et mélangé à 110°C avec 2,5 t/h d'un flux de monoéthylène glycol (MEG) comprenant 2,13 t/h de MEG provenant d'un bac de stockage et 0,37 t/h de MEG recyclé provenant de la section de purification du MEG.

Les quantités de PTA et MEG introduits correspondent à un ratio molaire PTA/MEG de 1,23. A 110°C, le taux volumique de solide, défini comme le ratio du volume de solide par le volume totale de la pâte (solide + liquide), est de 60,7 % volumique. Le mélange obtenu forme une pâte visqueuse.

Le mélange obtenu est ensuite transférée à l'aide d'une pompe adaptée vers un premier réacteur d'estérification opéré à 260°C, 5 bara (soit 0,5 MPa) avec un temps de séjour de 1,25 h.

1,4 t/h d'un effluent vapeur comprenant 71% poids d'eau et 29% poids de MEG est soutiré et envoyé dans une colonne de reflux pour séparer l'eau formée par la réaction d'estérification et le MEG. Ce dernier est ensuite renvoyé dans le réacteur. Une conversion du PTA de 85% est obtenue dans le premier réacteur.

L'effluent liquide du premier réacteur est envoyé ensuite dans un second réacteur d'estérification opéré à 260°C et 2 bara (soit 0,2 MPa) avec un temps de séjour de 1,25 h. 140 kg/h d'un effluent vapeur comprenant 40% poids d'eau et 60% poids de MEG est soutiré du second réacteur et envoyé à la colonne de reflux. Une conversion du PTA de 92% est atteinte à la sortie du second réacteur.

L'effluent liquide du second réacteur d'estérification est envoyé dans un troisième réacteur opéré à 275°C et 33 mbar (soit 0,033 MPa) avec un temps de séjour de 0,5 h qui permet de pousser la conversion du PTA à 95,8% et d'initier la polycondensation. Du trioxyde d'antimoine est ajouté comme catalyseur de polymérisation en entrée du troisième réacteur à hauteur de 220 ppm poids. Un effluent vapeur comprenant 70% poids de MEG, 16,5% poids d'eau, 5,5% poids d'acétaldéhyde, 2,5% poids de diéthylène glycol et 5,5% poids d'oligomères est soutiré du troisième réacteur et partiellement condensé puis envoyé à la section de purification du MEG.

L'effluent liquide du troisième réacteur est envoyé dans un quatrième réacteur (réacteur de polycondensation) opéré à 275°C et 66 mbar (soit 0,0066 MPa) avec un temps de séjour de 0,5 h. Un effluent vapeur de composition 60% poids de MEG, 25% poids d'eau, 6% poids d'acétaldéhyde, 3% poids de diéthylène glycol et 6% poids d'oligomères est soutiré du quatrième réacteur et partiellement condensé puis envoyé à la section de purification du MEG.

L'effluent liquide du quatrième réacteur est envoyé dans un dernier réacteur (réacteur de polycondensation) opéré à 280°C et 1,3 mbar (soit 0,000013 MPa) avec un temps de séjour de 1 h. Un effluent vapeur de composition 57% poids de MEG et 43% poids d'eau est soutiré et partiellement condensé puis envoyé à la section de purification du MEG.

La section de purification du MEG comprend une première colonne de distillation munie de 25 plateaux opérée en tête à 145°C et 200 mbar (soit 0,02 MPa) permettant de séparer le diéthylène glycol. Le fond de la première colonne de distillation est envoyé dans une seconde colonne de distillation munie de 17 plateaux opérée en tête à 100°C et 1 bara (soit 0,1 MPa) permettant de séparer les composés légers tel que l'eau et l'acétaldéhyde. Le MEG récupéré à l'issu de ces 2 distillations présente une pureté supérieure à 99,8% et est ensuite recyclé vers le ballon mélangeur.

6,25 t/h de PET sont produits. La consommation globale en énergie primaire de la production de PET est de 5,8 MMkcal/h.

Production de BHET solide décoloré et dépigmenté et incorporation d'au moins une fraction dans le procédé de polymérisation de PET
4 t/h de paillettes issues d'une charge PET à recycler, broyée et lavée, constituée à 50% poids de PET opaque et 50% poids de PET coloré, sont fondus dans une extrudeuse à 250 °C et mélangés avec 11,4 t/h d'éthylène glycol (MEG). Le mélange obtenu est injecté dans un réacteur agité, maintenu à 220°C et à une pression de 4 bara (soit 0,4MPa), pendant un temps de séjour de 4h. A la sortie du réacteur, l'effluent réactionnel comprend 66% poids de MEG, 27,4% poids de BHET, 1,7% poids de diéthylène glycol (DEG), 0,2% poids d'eau et 4,7% poids d'oligomères, pigments et autres composés lourds .

L'éthylène glycol présent dans l'effluent réactionnel est séparé par évaporation dans une succession de 5 ballons à des températures allant de 200°C à 124°C et des pressions de 0,1 MPa à 0,00025 MPa. A l'issue de cette étape d'évaporation, un flux de MEG de 10,95 t/h composé de 97% poids de MEG, 2,5% poids de DEG, 0,2% poids d'eau et 0,2% poids BHET, et un flux liquide riche en BHET de 5,17 t/h sont récupérés. Le flux de MEG est envoyé dans une première distillation munie de 25 plateaux et opérée en tête à 200 mbar (soit 0,02 MPa) et 145°C pour séparer le DEG et produits lourds puis dans une seconde colonne de distillation munie de 17 plateaux et opérée en tête à 100°C et 1 bara (soit 0,1 MPa) pour séparer l'eau et récupérer un effluent MEG purifié qui peut ensuite être recyclé vers le réacteur de dépolymérisation en mélange avec un appoint de MEG frais. Le flux liquide riche en BHET comprend 87,1% poids de BHET, 0,2% poids de MEG, 0,1% poids de DEG et 12,6% poids d'oligomères, pigments et autres composés lourds.

Le flux liquide riche en BHET est ensuite injecté dans une distillation court trajet à une température de 205°C et une pression de 0,2 mbar (soit 0,00002 MPa). Un effluent liquide de BHET pré-purifié avec un débit de 4,46 t/h est récupéré en refroidissant les vapeurs dans la distillation court-trajet à 115 °C. Il comprend 99,8% poids de BHET, 0,1 % poids de MEG et 0,1 % poids de DEG. Un résidu lourd comprenant 93% poids d'oligomères, pigments et autres composés lourds et 7% poids de BHET est également récupéré à un débit de 0,7 t/h à la sortie de la distillation court trajet.

Le flux liquide de BHET pré-purifié est comprimé jusqu'à 0,5 MPa et chauffé à 150°C puis alimente un lit fixe de charbon actif ayant une capacité d'adsorption égale à 5% de sa masse. A l'issue de cette étape, un flux liquide de BHET décoloré et dépigmenté est récupéré. Il est ensuite refroidi et solidifié à 40°C.

La consommation globale en énergie primaire de la dépolymérisation est de 1,25 MMkcal/t BHET.

Une fraction du BHET solide décoloré et dépigmenté est réinjecté dans une étape de préparation du mélange des monomères du procédé de polymérisation de PET similaire au procédé de polymérisation décrit ci-dessus en vue de produire 6,25 t/h de PET au total. Le procédé de polymérisation suivi est identique à celui décrit ci-dessus. Les étapes de dépolymérisation et de polymérisation sont complètement indépendantes et non intégrées.

Le tableau 1 ci-dessous reporte les quantités de monomères PTA, MEG et de monomère BHET solide incorporé, les taux de solide dans le mélange des charges obtenu à 110°C, le rapport du nombre de motif diol sur le nombre de motif téréphtalate et la consommation globale en énergie primaire pour la production de 6,25 t/h de PET tenant compte de l'incorporation de BHET issu du procédé de dépolymérisation décrit ci-dessus, pour deux rapports de motifs de diol sur les motifs de téréphtalate (1,23 et 1,1). Les résultats présentés sont des résultats calculés, à différentes quantités de MEG introduites, en considérant que 1 mol de BHET remplace dans le mélange 1 mol de PTA et 2 mol de MEG et basés sur des simulations procédés intégrant des données de solubilité et des données thermodynamiques calées sur des points expérimentaux.

| | | Exemple 1a | Exemple 1b |
|---|---|---|---|
| Quantité de PTA | [t/h] | 4,36 | 4,36 |
| Quantité de MEG (frais + recyclé) pour la polymérisation | [t/h] | 1,65 | 1,39 |
| Quantité de BHET solide incorporé | [t/h] | 1,74 | 1,74 |
| Rapport motif diol/motif téréphtalate | [mol/mol] | 1,23 | 1,1 |
| Taux de solide | [% vol] | 49,2 | 51,5 |
| Conso. énergie primaire - Etape de dépolymérisation | [MMkcal/h] | 2,17 | 2,17 |
| Conso. énergie primaire - Etape de polymérisation | [MMkcal/h] | 5,76 | 5,24 |
| Conso. énergie primaire totale | [MMkcal/h] | 7,93 | 7,41 |

### EXEMPLE 2 - Selon l'invention

Production de PET incorporant du PET à recycler avec intégration des procédés de dépolymérisation et de polymérisation

1,56 t/h de paillettes issues d'une charge PET à recycler, broyée et lavée, constituée à 50% poids de PET opaque et 50% poids de PET coloré, sont fondus dans une extrudeuse à 250 °C et mélangés à 4,68 t/h d'éthylène glycol (MEG). Le mélange est ensuite injecté dans un réacteur agité à 220°C et à une pression de 4 bara (soit 0,4 MPa). Le temps de séjour est fixé à 4 h. A la sortie du réacteur, l'effluent réactionnel comprend 66% poids de MEG, 27,4% poids de BHET, 1,7% poids de DEG, 0,2% poids d'eau et 4,7% poids d'oligomères, pigments et autres composés lourds .

L'éthylène glycol présent dans l'effluent réactionnel est séparé par évaporation dans une succession de 5 ballons opérés à des températures allant de 200°C à 124°C et des pressions de 0,1 MPa à 0,00025 MPa et est ensuite envoyé dans une de stripage et une colonne de rectification.

A l'issue de cette étape de séparation, un flux riche en MEG (comprenant 97% poids de MEG) et un flux liquide riche en BHET de 1,76 t/h sont récupérés. Le flux riche en MEG est fractionné : une partie est directement recyclée vers l'étape de dépolymérisation et l'étape de préparation des monomères en amont de l'étape d'estérification, une autre partie est envoyé vers une section de purification du MEG (décrite ci-dessous). Le flux liquide riche en BHET comprend 87,1% poids de BHET, 0,2 % poids de MEG et 0,1 % poids de DEG et 12,6 % poids d'oligomères, pigments et autres composés lourds.

Le flux liquide riche en BHET est ensuite injecté dans une distillation court trajet à une température de 205°C et une pression de 0,2 mbar (soit 0,00002 MPa). Un effluent liquide de BHET pré-purifié avec un débit de 1,74 t/h est récupéré en refroidissant les vapeurs dans la distillation court-trajet à 115 °C. Il comprend 99,8% poids de BHET, 0,1% poids de MEG et 0,1 % poids de DEG. Un résidu lourd avec un débit de 0,27 t/h est également récupéré à la sortie de la distillation court trajet : il comprend 93%poids d'oligomères, pigments et autres composés lourds et 7% poids de BHET.

Le flux liquide de BHET pré-purifié est comprimé jusqu'à 0,5 MPa et chauffé à 150°C puis alimente un lit fixe de charbon actif ayant une capacité d'adsorption égale à 5% de sa masse. A l'issue de cette étape, on obtient un flux liquide de BHET décoloré et dépigmenté, à une température d'environ 150°C. Ce flux liquide de BHET décoloré et dépigmenté est directement envoyé à l'étape de préparation des monomères du procédé de polymérisation.

Le flux liquide de BHET décoloré et dépigmenté est mélangé dans un ballon mélangeur à 110°C et équipé d'une agitation mécanique, avec :
- 4,36 t/h d'acide téréphtalique (PTA) et 1,65 t/h de monoéthylène glycol (MEG) (exemple 2a), ou
- 4,36 t/h d'acide téréphtalique (PTA) et 1,39 t/h de monoéthylène glycol (MEG) (exemple 2b). Le mélange obtenu sous forme de pâte est ensuite envoyé vers les étapes d'estérification et polycondensation comme celles décrites dans l'exemple 1.

Les condensats riche en MEG de la section de polycondensation sont envoyés dans à la section de purification du MEG. La section de purification du MEG est similaire à celle décrite dans l'exemple 1. Le MEG récupéré à l'issue de la section de purification du MEG présente une pureté supérieure à 99,8%.

Le tableau 2 ci-dessous reporte la quantité de monomères PTA, MEG frais et de PET à recycler pour produire 6,25 t/h de PET, le taux de solide obtenu à l'étape de préparation des charges monomères, le rapport du nombre de motif diol sur le nombre de motif téréphtalate dans ladite étape et la consommation globale en énergie primaire pour le procédé intégré. Les résultats présentés sont des résultats calculés en considérant que 1 mol de BHET remplace dans le mélange 1 mol de PTA et 2 mol de MEG et basés sur des simulations procédés intégrant des données de solubilité et des données thermodynamiques calées sur des points expérimentaux.

| | | Exemple 2a | Exemple 2b |
|---|---|---|---|
| Quantité de PTA | [t/h] | 4,36 | 4,36 |
| Quantité de MEG frais | [t/h] | 1,17 | 1,17 |
| Quantité de rPET | [t/h] | 1,56 | 1,56 |
| Rapport motif diol/motif téréphtalate dans l'étape de préparation des monomères | [mol/mol] | 1,23 | 1,1 |
| Taux de solide dans l'étape de préparation des monomères | [% vol] | 49,2 | 51,5 |
| Conso. énerqie primaire totale | [MMkcal/h] | 7,45 | 6,9 |

Il apparait clairement qu'à rapport équivalent de nombre de motifs diol sur le nombre de motifs téréphtalate introduits dans l'étape de préparation de la charge de polymérisation (étape de préparation des monomères), le procédé global qui intègre des étapes de dépolymérisation et des étape de polymérisation présente un gain en consommations énergétiques par rapport à la somme des consommations énergétiques d'un procédé de dépolymérisation et d'un procédé de polymérisation non intégrés :
- pour un rapport de motifs diol/téréphtalate de 1,23 : gain de 0,48 MMkcal/h (soit environ 6%) ;
- pour un rapport de motifs diol/téréphtalate1,1 : gain de 0,51 MMkcal/h (soit environ 7%).

## Revendications

1. Procédé de production d'un polyester téréphtalate à partir d'au moins une charge polyester à recycler comprenant au moins 10% poids de PET opaque, comprenant au moins les étapes suivantes :
a) une étape de dépolymérisation de ladite charge polyester à recycler, comprenant au moins une section réactionnelle alimentée en ladite charge polyester à recycler et en une charge glycol comprenant au moins une fraction d'un flux diol purifié, ladite section réactionnelle étant opérée à une température comprise entre 150 et 400°C, de préférence entre 180 et 300°C, de manière préférée entre 200°C et 280°C, à une pression d'au moins 0,1 MPa, préférentiellement au moins 0,4 MPa, et avec un temps de séjour par réacteur compris entre 0,05 et 10 h, pour obtenir un effluent réactionnel de dépolymérisation,
b) une étape de séparation, comprenant au moins une section de séparation alimentée en ledit effluent réactionnel de dépolymérisation obtenu à l'issue de l'étape a) de dépolymérisation, pour obtenir au moins un effluent glycol et un effluent diester,
c) une étape de purification de l'effluent diester obtenu à l'issue de l'étape b), comprenant au moins une section de séparation alimentée par ledit effluent diester obtenu à l'issue de l'étape b) et opérée à une température inférieure ou égale à 250°C, à une pression inférieure ou égale à 0,001 MPa, et avec un temps de séjour liquide par section inférieur ou égal à 10 min, puis une section de décoloration opérée à une température entre 100 et 250°C et à une pression entre 0,1 et 1,0 MPa, en présence d'un adsorbant, pour obtenir un effluent diester purifié liquide comprenant au moins du bis(2-hydroxyéthyl) téréphtalate (BHET),
d) une étape de préparation d'une charge de polymérisation comprenant au moins une section de mélange alimentée en au moins une charge téréphtalique, qui comprend de l'acide téréphtalique ou du diméthyle téréphtalate, et au moins une fraction dudit effluent diester purifié liquide obtenu à l'étape c), les quantités en au moins ladite charge téréphtalique et ladite fraction de l'effluent diester purifié liquide, introduites dans ladite section de mélange, étant ajustées de sorte que le rapport du nombre total de moles de motifs diol de formule -[C₍ₙ₊₁₎H₍₂ₙ₊₂₎O₂]-, n étant un nombre entier supérieur ou égal à 1, introduits dans ladite section de mélange, par rapport au nombre total de moles de motifs téréphtalate de formule -[CO-(C₆H₄)-CO]-, introduits dans ladite section de mélange, est compris entre 1,0 et 2,0, ladite section de mélange étant opérée à une température comprise entre 100 et 150°C et à une pression supérieure ou égale à 0,1 MPa,
e) une étape de condensation de ladite charge de polymérisation issue de l'étape d), pour produire au moins un effluent réactionnel de condensation, un effluent diol et un effluent aqueux ou un effluent méthanol, ladite étape de condensation comprenant au moins une section réactionnelle opérée à une température entre 150 et 400°C, à une pression entre 0,05 et 1 MPa, et avec un temps de séjour entre 0,5 et 10 h, et au moins une section de séparation,
f) une étape de polycondensation dudit effluent réactionnel de condensation obtenu à l'étape e) pour obtenir au moins ledit polyester téréphtalate et un effluent diol, ladite étape de polycondensation comprenant au moins une section réactionnelle comprenant au moins un réacteur dans lequel est mise en oeuvre la polycondensation et étant opérée à une température entre 200 et 400°C, à une pression entre 0,0001 et 0,1 MPa, avec un temps de séjour entre 0,1 et 5 h, ladite section réactionnelle comprenant également au moins un soutirage dudit effluent diol,
g) une étape de traitement des diols, comprenant une section de récupération alimentée au moins par tout ou partie de l'effluent glycol issu de l'étape b) et tout ou partie de l'effluent diol issu de l'étape f), pour obtenir un flux diol à traiter, et une section de purification dudit flux diol à traiter pour obtenir le flux diol purifié qui est envoyé en tout ou partie vers l'étape a).

2. Procédé de production selon la revendication 1, dans lequel ladite charge polyester à recycler comprend au moins 15% poids de PET opaque.

3. Procédé de production selon la revendication 1 ou 2, dans lequel ladite charge polyester à recycler comprend entre 0,1% et 10% poids de pigments, notamment entre 0,1 et 5% poids de pigments de pigments.

4. Procédé de production selon l'une des revendications précédentes, dans lequel la section réactionnelle de ladite étape a) est alimentée en ladite charge polyester à recycler et en ladite charge glycol de sorte que la quantité du composé glycol contenu dans ladite charge glycol correspond, en entrée de ladite section de réactionnelle, à 1 à 20 moles, de préférence de 3 à 10 moles, de composé glycol par mole de motif élémentaire de répétition du polyester contenu dans ladite charge polyester à recycler.

5. Procédé de production selon l'une des revendications précédentes, dans lequel la section de séparation de l'étape b) comprend une succession de 1 à 5 séparations gaz-liquide, opérées à une température comprise entre 100 et 250°C, et à une pression comprise entre 0,00001 et 0,2 MPa.

6. Procédé de production selon l'une des revendications précédentes, dans lequel la section de séparation de l'étape c) de purification met en oeuvre un système d'évaporation à film tombant ou à film raclé, par distillation à court trajet à film tombant ou à film raclé, ou par une succession de plusieurs évaporations et/ou distillations court trajet à film tombant ou film raclé, à une température inférieure ou égale à 250°C, de manière préférée inférieure ou égale à 230 °C, de manière très préférée inférieure ou égale à 200°C, et à une pression inférieure ou égale à 0,001 MPa, de préférence inférieure ou égale à 0,0001 MPa, de manière préférée inférieure ou égale à 0,00005 MPa.

7. Procédé de production selon l'une des revendications précédentes, dans lequel l'adsorbant de la section de décoloration de l'étape c) est un charbon actif.

8. Procédé de production selon l'une des revendications précédentes, dans lequel les quantités en charge téréphtalique, et en effluent diester purifié liquide, introduites dans ladite section de mélange de l'étape d) sont ajustées de sorte que le rapport du nombre total de moles de motifs diol introduits dans ladite section de mélange, par rapport au nombre total de moles de motifs téréphtalate introduits dans ladite section de mélange, est compris entre 1,0 et 1,5, préférentiellement entre 1,0 et 1,3.

## Patentansprüche

1. Verfahren zur Herstellung eines Polyesterterephthalats aus mindestens einer zu recycelnden Polyestercharge, die mindestens 10 Gew.-% undurchsichtiges PET umfasst, umfassend mindestens die folgenden Schritte:
a) einen Schritt der Depolymerisation der zu recycelnden Polyestercharge, umfassend mindestens einen Reaktionsabschnitt, der mit der zu recycelnden Polyestercharge und mit einer Glykolcharge, die mindestens eine Fraktion eines gereinigten Diolstroms umfasst, beschickt wird, wobei der Reaktionsabschnitt bei einer Temperatur zwischen 150 und 400 °C, bevorzugt zwischen 180 und 300 °C, besonders bevorzugt zwischen 200 °C und 280°C, bei einem Druck von mindestens 0,1 MPa, bevorzugt mindestens 0,4 MPa, und mit einer Verweilzeit je Reaktor zwischen 0,05 und 10 h betrieben wird, um einen Depolymerisationsreaktionsabstrom zu erhalten,
b) einen Schritt des Trennens, umfassend mindestens einen Trennabschnitt, der mit dem nach dem Schritt der Depolymerisation a) erhaltenen Depolymerisationsreaktionsabstrom beschickt wird, um mindestens einen Glycolabstrom und einen Diesterabstrom zu erhalten,
c) einen Schritt des Reinigens des nach dem Schritt b) erhaltenen Diesterabstroms, umfassend mindestens einen Trennabschnitt, der mit dem nach dem Schritt b) erhaltenen Diesterabstrom beschickt wird und bei einer Temperatur kleiner oder gleich 250 °C, bei einem Druck kleiner oder gleich 0,001 MPa und mit einer Flüssigverweilzeit je Abschnitt kleiner oder gleich 10 min betrieben wird, dann einen Entfärbungsabschnitt, der bei einer Temperatur zwischen 100 und 250 °C und bei einem Druck zwischen 0,1 und 1,0 MPa in Gegenwart eines Adsorbens betrieben wird, um einen flüssigen gereinigten Diesterabstrom zu erhalten, der mindestens Bis(2-hydroxyethyl)terephtalat (BHET) umfasst,
d) einen Schritt des Zubereitens einer Polymerisationscharge, umfassend mindestens einen Mischabschnitt, der mit mindestens einer terephthalischen Charge, die Terephthalsäure oder Dimethylterephtalat umfasst, und mindestens einer Fraktion des im Schritt c) erhaltenen flüssigen gereinigten Diesterabstroms beschickt wird, wobei die Mengen an mindestens der terephthalischen Charge und der Fraktion des flüssigen gereinigten Diesterabstroms, die in den Mischabschnitt eingeleitet werden, so angepasst sind, dass das Verhältnis der Gesamtmolzahl der Dioleinheiten mit der Formel - [C₍ₙ₊₁₎H₍₂ₙ₊₂₎O₂]-, wobei n eine ganze Zahl grösser oder gleich 1 ist, die in den Mischabschnitt eingeleitet werden, zur Gesamtmolzahl von Terephthalateinheiten mit der Formel -[CO-(C₆H₄)-CO]-, die in den Mischabschnitt eingeleitet werden, zwischen 1,0 und 2,0 beträgt, wobei der Mischabschnitt bei einer Temperatur zwischen 100 und 150 °C und bei einem Druck größer oder gleich 0,1 MPa betrieben wird,
e) einen Schritt der Kondensation der aus dem Schritt d) hervorgegangenen Polymerisationscharge, um mindestens einen Kondensationsreaktionsabstrom, einen Diolabstrom und einen wässrigen Abstrom oder einen Methanolabstrom herzustellen, wobei der Schritt der Kondensation mindestens einen Reaktionsabschnitt, der bei einer Temperatur zwischen 150 und 400 °C, bei einem Druck zwischen 0,05 und 1 MPa und mit einer Verweilzeit zwischen 0,5 und 10 h betrieben wird, und mindestens einen Trennabschnitt umfasst,
f) einen Schritt der Polykondensation des im Schritt e) erhaltenen Kondensationsreaktionsabstroms, um mindestens das Polyesterterephthalat und einen Diolabstrom zu erhalten, wobei der Schritt der Polykondensation mindestens einen Reaktionsabschnitt umfasst, der mindestens einen Reaktor umfasst, in dem die Polykondensation durchgeführt wird und der bei einer Temperatur zwischen 200 und 400 °C, bei einem Druck zwischen 0,0001 und 0,1 MPa, mit einer Verweilzeit zwischen 0,1 und 5 h betrieben wird, wobei der Reaktionsabschnitt auch mindestens ein Abziehen des Diolabstroms umfasst,
g) einen Schritt des Behandelns der Diole, umfassend einen Rückgewinnungsabschnitt, der mit mindestens dem gesamten oder einem Teil des aus dem Schritt b) hervorgegangenen Glycolabstroms und dem gesamten oder einem Teil des aus dem Schritt f) hervorgegangenen Diolabstroms beschickt wird, um einen zu behandelnden Diolstrom zu erhalten, und einen Abschnitt zum Reinigen des zu behandelnden Diolstroms, um den gereinigten Diolstrom zu erhalten, der vollständig oder teilweise zu dem Schritt a) geleitet wird.

2. Verfahren zur Herstellung nach Anspruch 1, bei dem die zu recycelnde Polyestercharge mindestens 15 Gew.-% undurchsichtiges PET umfasst.

3. Verfahren zur Herstellung nach Anspruch 1 oder 2, bei dem die zu recycelnde Polyestercharge zwischen 0,1 und 10 Gew.-% Pigmente, insbesondere zwischen 0,1 und 5 Gew.-% Pigmente umfasst.

4. Verfahren zur Herstellung nach einem der vorhergehenden Ansprüche, bei dem der Reaktionsabschnitt des Schritts a) mit der zu recycelnden Polyestercharge und mit der Glycolcharge so beschickt wird, dass die Menge der in der Glycolcharge enthaltenen Glycolverbindung im Einlass des Reaktionsabschnitts 1 bis 20 Mol, bevorzugt 3 bis 10 Mol, Glycolverbindung je Mol elementare Wiederholungseinheit des in der zu recycelnden Polyestercharge enthaltenen Polyesters entspricht.

5. Verfahren zur Herstellung nach einem der vorhergehenden Ansprüche, bei dem der Trennabschnitt des Schritts b) eine Abfolge von 1 bis 5 Gas-Flüssig-Trennungen umfasst, die bei einer Temperatur zwischen 100 und 250 °C und bei einem Druck zwischen 0,00001 und 0,2 MPa durchgeführt werden.

6. Verfahren zur Herstellung nach einem der vorhergehenden Ansprüche, bei dem der Trennabschnitt des Schritts c) des Reinigens ein Fallfilm- oder Wischfilmverdampfersystem durch Kurzweg-Fallfilm- oder - Wischfilmdestillation oder durch eine Abfolge von mehreren Kurzweg-Fallfilm- oder -Wischfilmverdampfungen und/oder -destillationen bei einer Temperatur kleiner oder gleich 250 °C, bevorzugt kleiner oder gleich 230 °C, besonders bevorzugt kleiner oder gleich 200 °C und bei einem Druck kleiner oder gleich 0,001 MPa, bevorzugt kleiner oder gleich 0,0001 MPa, besonders bevorzugt kleiner oder gleich 0,00005 MPa einsetzt.

7. Verfahren zur Herstellung nach einem der vorhergehenden Ansprüche, bei dem das Adsorbens des Entfärbungsabschnitts des Schritts c) eine Aktivkohle ist.

8. Verfahren zur Herstellung nach einem der vorhergehenden Ansprüche, bei dem die Mengen an terephthalischer Charge und an flüssigem gereinigtem Diesterabstrom, die in den Mischabschnitt des Schritts d) eingeleitet werden, so angepasst sind, dass das Verhältnis der Gesamtmolzahl von Dioleinheiten, die in den Mischabschnitt eingeleitet werden, zu der Gesamtmolzahl von Terephthalateinheiten, die in den Mischabschnitt eingeleitet werden, zwischen 1,0 und 1,5, bevorzugt zwischen 1,0 und 1,3 beträgt.

## Claims

1. Process for producing a terephthalate polyester from at least one feedstock of polyester to be recycled comprising at least 10% by weight of opaque PET, said process comprising at least the following steps:
a) a step of depolymerization of said feedstock of polyester to be recycled, comprising at least one reaction section fed with said feedstock of polyester to be recycled and with a glycol feedstock comprising at least a fraction of the purified diol stream, said reaction section being operated, at a temperature between 150°C and 400°C, preferably between 180°C and 300°C, preferably between 200°C and 280°C, at a pressure of at least 0.1 MPa, preferentially at least 0.4 MPa, and with a residence time per reactor of between 0.05 and 10 h, in order to obtain a depolymerization reaction effluent,
b) a separation step, comprising at least one separation section fed with said depolymerization reaction effluent obtained at the end of the depolymerization step a), in order to obtain at least one glycol effluent and one diester effluent,
c) a step of purification of the diester effluent obtained at the end of step b), comprising at least one separation section fed with said diester effluent obtained at the end of step b) and operated at a temperature below or equal to 250°C, at a pressure less than or equal to 0.001 MPa, and with a liquid residence time per section of the less than equal to 10 min, then a bleaching section operated at a temperature between 100°C and 250°C and at a pressure of between 0.1 and 1.0 MPa, in the presence of an adsorbent, in order to obtain a liquid purified diester effluent comprising at least bis(2-hydroxyethyl) terephthalate (BHET),
d) a step of preparation of a polymerization feedstock comprising at least one mixing section fed with at least one terephthalic feedstock, which comprises terephthalic acid or dimethyl terephthalate, and at least one fraction of said liquid purified diester effluent obtained in step c), the amounts of at least said terephthalic feedstock and said fraction of the liquid purified diester effluent, introduced into said mixing section, being adjusted so that the ratio of the total number of moles of diol units of formula - [C₍ₙ₊₁₎H₍₂ₙ₊₂₎O₂]-, n being an integer greater than or equal to 1, introduced into said mixing section, relative to the total number of moles of terephthalate units of formula -[CO-(C₆H₄)-CO]-, introduced into said mixing section, is between 1.0 and 2.0, said mixing section being operated at a temperature between 100°C and 150°C and at a pressure greater than or equal to 0.1 MPa,
e) a step of condensation of said polymerization feedstock resulting from step d), in order to produce at least one condensation reaction effluent, one diol effluent and one aqueous effluent or one methanol effluent, said condensation step comprising at least one reaction section operated at a temperature between 150°C and 400°C, at a pressure between 0.05 and 1 MPa, and with a residence time of between 0.5 and 10 h, and at least one separation section,
f) a step of polycondensation of said condensation reaction effluent obtained in step e) in order to obtain at least said terephthalate polyester and a diol effluent, said polycondensation step comprising at least one reaction section comprising at least one reactor in which the polycondensation is carried out and being operated at a temperature between 200°C and 400°C, at a pressure between 0.0001 and 0.1 MPa, with a residence time between 0.1 and 5 h, said reaction section also comprising at least one drawing-off of said diol effluent,
g) a step of treating the diols, comprising a recovery section fed at least by all or part of the glycol effluent resulting from step b) and all or part of the diol effluent resulting from step f), in order to obtain a diol stream to be treated, and a section for purification of said diol stream to be treated in order to obtain a purified diol stream which is sent, totally or partly, to step a).

2. Production process according to Claim 1, in which said feedstock of polyester to be recycled comprises at least 15% by weight of opaque PET.

3. Production process according to Claim 1 or 2, in which said feedstock of polyester to be recycled comprises 0.1% and 10% by weight of pigments, notably between 0.1% and 5% by weight of pigments.

4. Production process according to one of the preceding claims, in which the reaction section of step a) is fed with said feedstock of polyester to be recycled and with said glycol feedstock so that the amount of the glycol compound contained in said glycol feedstock corresponds, at the inlet of said reaction section, to 1 to 20 mol, preferably 3 to 10 mol, of glycol compound per mole of basic repeat unit of the polyester contained in said feedstock of polyester to be recycled.

5. Production process according to one of the preceding claims, in which the separation section of step b) comprises a succession of 1 to 5 gas-liquid separations, carried out at a temperature of between 100°C and 250°C and at a pressure of between 0.00001 and 0.2 MPa.

6. Production process according to one of the preceding claims, in which the separation section of the purification step c) uses a system of falling film or wiped film evaporation, by falling film or wiped film short path distillation, or by a succession of several falling film or wiped film short path distillations and/or evaporations, at a temperature below or equal to 250°C, preferably below or equal to 230°C, very preferably below or equal to 200°C, and at a pressure less than or equal to 0.001 MPa, preferably less than or equal to 0.0001 MPa, preferably less than or equal to 0.00005 MPa.

7. Production process according to one of the preceding claims, in which the adsorbent of the bleaching section of step c) is an activated carbon.

8. Production process according to one of the preceding claims, in which the amounts of terephthalate feedstock, and of liquid purified diester effluent, introduced into said mixing section of step d) are adjusted so that the ratio of the total number of moles of diol units introduced into said mixing section, relative to the total number of moles of terephthalate units introduced into said mixing section, is between 1.0 and 1.5, preferentially between 1.0 and 1.3.
